# EUROPÄISCHE PATENTSCHRIFT

(11) **EP 1 175 435 B1**
(45) Veröffentlichungstag und Bekanntmachung des Hinweises auf die Patenterteilung: **20.04.2005**
(21) Anmeldenummer: 00936703.8
(22) Anmeldetag: 29.04.2000
(51) Int. Cl.: C07K 7/08, A61K 38/10, A61P 31/00, C12P 21/02

(54) **CEPHAIBOLE, ANTIPARASITIKA AUS ACREMONIUM TUBAKII, VERFAHREN ZU IHRER HERSTELLUNG UND VERWENDUNG DERSELBEN**
CEPHAIBOLS, NOVEL ANTIPARASITICS FROM ACREMONIUM TUBAKII, METHOD FOR THE PRODUCTION AND THE UTILIZATION THEREOF
CEPHAIBOLS, NOUVEAUX ANTIPARASITES OBTENUS A PARTIR DE ACREMONIUM TUBAKII, LEUR PROCEDE DE PRODUCTION ET LEUR UTILISATION

(30) Priorität: 05.05.1999 DE 19920816
(43) Veröffentlichungstag der Anmeldung: 30.01.2002
(73) Patentinhaber: Aventis Pharma Deutschland GmbH, 65929 Frankfurt am Main (DE)
(72) Erfinder: VERTESY, Laszlo, D-65817 Eppstein (DE); KURZ, Michael, D-65719 Hofheim (DE); SCHIELL, Matthias, D-65611 Brechen (DE); HOFMANN, Joachim, D-65520 Bad Camberg (DE)
(86) Internationale Anmeldenummer: PCT/EP2000/003894
(87) Internationale Veröffentlichungsnummer: WO 2000/068256

(56) Entgegenhaltungen:
- US-A- 3 657 419
- PANDEY E.A.: "Structure of the peptide antibiotic Antiamoebin II" JOURNAL OF ANTIBIOTICS., Bd. XXXI, Nr. 3, März 1978 (1978-03), Seiten 241-243, XP002149655 JAPAN ANTIBIOTICS RESEARCH ASSOCIATION. TOKYO., JP ISSN: 0021-8820
- RINEHART K: "Fast atom bombardment mass spectrometry: a promising tool for structural studies" TRENDS IN ANALYTICAL CHEMISTRY, Bd. 2, Nr. 1, 1983, Seiten 10-14, XP000952232
- SHARMAN, GARY J. ET AL: "Structural elucidation of XR586, a peptaibol -like antibiotic from Acremonium persicinum" BIOCHEM. J. (1996), 320(3), 723-728 , XP002149656
- JAWORSKI, ANDREAS ET AL: "New sequences and new fungal producers of peptaibol antibiotics antiamoebins" J. PEPT. SCI., Bd. 6, Nr. 4, April 2000 (2000-04), Seiten 149-167, XP000951583

## Beschreibung

Die Erfindung betrifft neue Peptaibol-Antibiotika, im folgenden Cephaibole genannt, die von Acremonium tubakii FH 1685 DSM 12774 während der Fermentation synthetisiert und in das Kulturmedium abgegeben werden, ein Verfahren zur Isolierung der Cephaibole aus dem Kulturmedium und ihre Aufreinigung, sowie die Verwendung der Cephaibole als pharmakologisch aktive Wirkstoffe, insbesondere zur Bekämpfung von Parasiten.

Parasitosen sind weitverbreitet und verursachen in Mensch und Tier ein weites Spektrum pathologischer Effekte, die von geringen physiologischen Störungen bis zu starken, sogar tödlichen Erkrankungen reichen. Heutzutage sind viele, intensiv erforschte Parasitenerkrankungen bekannt, die die Gesundheit und das Leben des Menschen und seiner Haus- und Nutztiere bedrohen.

Weltweit ist mit zunehmender Tendenz bei Millionen von Menschen eine Schwächung des Immunsystems festzustellen. Dieser Personenkreis wird von opportunistischen Parasiten so massiv befallen, daß jährlich Millionen von Todesopfem zu beklagen sind. Im Zeitalter der Femreisen muß auch in Nicht-Drittländem, die normalerweise einen hohen Hygienestandard besitzen, mit exotischen Parasiten gerechnet werden. Dies hat seinen Grund darin, daß ein immer stärkerer Trend zu Trekkingreisen unter landesüblichen Hygienebedingungen besteht und daß - aus der vermeintlichen Sicherheit im eigenen Land - ein Verlust des Gefühls / Wissens um Hygienegefahren entstanden ist. Neben dem Schutz der menschlichen Gesundheit verlangt auch der Tierschutz, durch Parasitosen hervorgerufene Leiden und Schmerzen zu heilen und möglichst abzuwenden. Wirtschaftliche Gründe kommen vor allem in der Nutztierhaltung zum Tragen, wo durch ungünstige Bedingungen der Haltung und Fütterung von Tieren (z.B. bei bestimmten Formen der Massentierhaltung) parasitäre Erkrankungen bevorzugt auftreten, die zur Leistungsminderung quantitativer (Fleischmenge, Eizahl, Rennzeit) oder qualitativer Art (Fleisch- und Wotlqualität) beitragen. Die großen Schäden, die durch Parasitosen in Mensch und Tier hervorgerufen werden, machen ihre Kontrolle wünschenswert wenn nicht unerläßlich im Interesse der Gesundheit und Wirtschaftlichkeit.

Parasiten sind einzellige oder mehrzellige Schmarotzer, die sich vorübergehend oder ständig in (Endoparasiten) oder an (Ektoparasiten) fremden Organismen aufhalten und sich zu Lasten des Wirtes ernähren. Viele Parasiten führen bei Mensch und Tier zu subakuten aber auch zu dramatischen Erkrankungen. Zu den einzelligen Parasiten zählt man die Protozoen, wie zum Beispiel Plasmodien (Erreger der Malaria), Trypanosomen (Erreger der Chagas-Krankheit), Amöben, Trichomonaden oder Toxoplasmen. Wichtige mehrzellige Parasiten sind die endoparasitischen Würmer (Helminthen), von denen die Fadenwürmer (Nematoden), Bandwürmer (Cestoden) und Egel (Trematoden) bei Mensch und Tier schwere Schäden verursachen können. Die ebenfalls mehrzelligen Ektoparasiten umfassen Zecken, Milben, Läuse, Flöhe und andere Organismen. Es steht zwar eine große Zahl von Mitteln zur Bekämpfung und Behandlung von parasitischen Erkrankungen (Antiparasitika) zur Verfügung, aber zum einen wegen der Nebenwirkungen dieser Agentien und zum anderen wegen der zunehmenden Resistenzbildung gibt es einen großen Bedarf an neuen, hoch wirksamen Protozooziden, Anthelminthika und anderen Parasitiziden. Der Befall mit Parasiten betrifft insbesondere die Bevölkerung der tropischen Regionen, hier wird die Zahl der Betroffenen auf viele hundert Millionen Menschen geschätzt, hinzu kommt der beträchtliche Schaden im landwirtschaftlichem Bereich.

Der Einsatz chemischer, in ihrer Wirksamkeit gegen einzelne Parasiten oder größere Parasitengruppen bekannter und im Wirt (Mensch, Tier) toxikologisch unbedenklicher Substanzen, hat in der Parasitenbekämpfung noch eine überragende Bedeutung.

Nach ihrem Wirkungsspektrum unterscheidet man gegen Helminthen wirkende Anthelminthika, gegen Protozoen wirksame Antiprotozoika, gegen Insekten wirksame Insektizide, gegen Milben (Akaria) wirksame Akarizide; die letzten beiden Gruppen faßt man auch unter dem Begriff Ektoparasitizide zusammen.

Die Zunahme von Arzneimittelresistenzen, begünstigt durch langjährigen und intensiven Einsatz besonders in der modernen Massentierhaltung oder das Auftreten von zum Teil starken Nebenwirkungen insbesondere bei Dauermedikation von Menschen, die im Rahmen fortschreitender Globalisierung längere Zeit in den Tropen- und Subtropen arbeiten müssen sowie hohe Kosten bei der Prophylaxe/Therapie mit bestimmten Chemotherapeutika macht die Suche nach preisgünstigen anderen Substanzklassen mit einem anderen Wirkmechanismus und besserer Verträglichkeit unumgänglich. Auch werden Antiparasitika benötigt, die nicht nur wirksam, sondern auch in großen Mengen kostengünstig herstellbar und zudem umweltfreundlich sind.

Peptide mit bis zu 20, zum Teil strukturell ungewöhnlichen Aminosäuren werden von Bakterien und Pilzen über deren Sekundärstoffwechsel mittels nichtribosomaler Peptid-Synthetases produziert. Viele der bisher bekannten Sekundärmetabolite mit Peptidstruktur besitzen interessante biologische Wirkungen als Antibiotika, Enzyminhibitoren, Kardiotonika, Immunmodulatoren, Insektizide, Nematozide u.a.m. (siehe z.B. Gräfe, U. Biochemie der Antibiotika, Spektrum Heidelberg, 1992).

Innerhalb der Strukturklasse der Peptidwirkstoffe sind die sogenannten Peptaibole dadurch ausgezeichnet, daß sie ungewöhnlich viele Aminosäuren (bis zu 20, darunter einen hohen Anteil an alpha-Aminobuttersäure enthalten (Brückner, H. , König, W.A., Greiner, M., Jung, G. Angew. Chem. Int. Ed. Engl. 18 (1979), 476 - 477). Ferner sind Peptaibole sehr häufig am N-Terminus acetyliert und weisen am C-Terminus einen Rest mit einer Alkoholgruppe (z.B. Phenylalaninol) oder einer Aldehydgruppe auf.

Beispiele für Peptaibole, wie oben angesprochen, sind Aibellin [J. Antibiotics, 47, S. 1136-1144(1994)]; Ampullosporin [J. Antibiotics, 50, S. 72 -728, (1997)]; Antiamoebin [Structure, 6, S. 783-792 (1998)]; Clonostachin [J. Antibiotics, 50, S. 105-110 (1997)]; Emerimicine [J. Antibiotics, 27, S. 274-282 (1974)] oder Zervamicine [J. Antibiotics, 27, S. 321-328 (1974)]. Diese Peptaibole werden von sehr verschiedenen Stämmen der Gattungen Emericellopsis, Trichoderma, Apiocrea und vielen anderen synthetisiert. Ihre antibiotische Wirksamkeit entfalten sie gegen gram-positive Bakterien, gegen einige Pilzarten und gegen Amöben. Darüber hinaus ist die fiebersenkende sowie neuroleptische Wirkung des Ampullosporins beschrieben worden.

In J. Antiobotics, 31, 241-243 (1978) und US 3,657,419 werden Peptaibole (Antiamoebin) mit antibiotischer Wirkung beschrieben.

Die Isolierung eines Peptaibol-ähnlichen Antibiotikums nach Fermentation eines Mikroorganismus wird in Biochem. J. 320, 723-728 (1996) beschrieben.

Die Strukturaufklärung eines weiteren Peptaibols wird in Trends in Analytical Chemistry, 2, 10-14 (1983) beschrieben.

Die bisher bekannten Peptidwirkstoffe weisen allerdings oft Nachteile auf, die sich in unbefriedigender Wirkhöhe, hoher Toxizität und/oder unerwünschten Nebenwirkungen äußern.

Aufgabe der Erfindung ist es daher, nach neuen mikrobiellen Peptidwirkstoffen mit verbesserten Eigenschaften und/oder neuen Wirkmechanismen zu suchen.

Diese Aufgabe wird erfindungsgemäß gelöst, indem man den Stamm Acremonium tubakii FH 1685 DSM 12774 in einer Nährlösung mit Kohlenstoff- und Stickstoffquelle sowie den üblichen anorganischen Salzen fermentiert, bis sich die die neuen Peptaibole, im folgenden Cephaibole genannt, in dem Kulturmedium anhäufen, anschließend die Cephaibole aus dem Kulturmedium isoliert und gegebenenfalls die Cephaibole in die einzelnen aktiven Peptidverbindungen auftrennt. Die isolierten Cephaibole sind pharmakologisch aktiv und eignen sich somit zur Verwendung als Arzneimittel. Aufgrund ihrer antiparasitischen Eigenschaften, insbesondere ihrer stark antihelmintischen Aktivität sowie einer insektiziden Wirkung auf Ektoparasiten können sie insbesondere als Antibiotika mit Wirkung gegen Endo- und Ektoparasiten bei Tieren sowie beim Menschen eingesetzt werden.

Die Erfindung betrifft somit
1. Eine Verbindung der allgemeinen Formel I in der R Phe-ol bedeutet und w, x, y, und z folgende Bedeutung haben:
   a) w gleich Gly oder Ala; x gleich Aib und y und z gleich Iva;
   b) w gleich Gly; x, y und z gleich Iva;
   c) w gleich Gly; x und z gleich Aib und y gleich Iva;
   d) w gleich Gly; x, y und z gleich Aib; oder
   e) w gleich Gly; x und y gleich Aib und z gleich Iva;
   sowie deren physiologisch verträglichen Salze; oder
   eine Verbindung der allgemeinen Formel II in der x Hyp oder Pro bedeutet; sowie deren physiologisch verträglichen Salze.
2. Ein Verfahren zur Herstellung einer oder mehrerer Verbindungen der allgemeinen Formel I oder II, das dadurch gekennzeichnet ist, daß man Acremonium tubakii, insbesonders Acremonium tubakii FH 1685 DSM 12774 in einem Kulturmedium fermentiert, bis sich eine oder mehrere Verbindungen der allgemeinen Formel I oder II in dem Kulturmedium anhäufen und man diese aus dem Kulturmedium isoliert.
3. Eine Verwendung einer Verbindung der allgemeinen Formel I oder II als pharmakologisch wirksamen Stoff, insbesondere als Antibiotikum gegen menschliche oder tierische Parasiten, besonders bevorzugt gegen Helminthen.
4. Eine pharmazeutische Zubereitung zur Verwendung bei Menschen und/oder bei Tieren, enthaltend eine oder mehrere Verbindungen der allgemeinen Formel I oder II.

Im folgenden wird die Erfindung detailliert beschrieben, insbesondere in ihren bevorzugten Ausführungsformen.

Verbindungen der allgemeinen Formel I oder 11 werden auch als Peptidwirkstoffe oder Cephaibole bezeichnet.

Cephaibol A bezeichnet eine Verbindung der allgemeinen Formel I, in der R Phe-ol, w Gly, x Aib und y und z Iva bedeutet.

Cephaibol A1 bezeichnet eine Verbindung der allgemeinen Formel I, in der R Phe-ol, w Ala, x Aib und y und z Iva bedeutet.

Cephaibol B bezeichnet eine Verbindung der allgemeinen Formel I, in der R Phe-ol, w Ala und x,y und z Iva bedeutet.

Cephaibol C bezeichnet eine Verbindung der allgemeinen Formel I, in der R Phe-ol, w Ala, x und z Aib und y Iva bedeutet.

Cephaibol D bezeichnet eine Verbindung der allgemeinen Formel I, in der R Phe-ol, w Ala und x, y und z Aib bedeutet.

Cephaibol E bezeichnet eine Verbindung der allgemeinen Formel I, in der R Phe-ol, w Ala, x und y Aib und z Iva bedeutet.

Cephaibol P bezeichnet eine Verbindung der allgemeinen Formel II, in der x Hyp bedeutet.

Cephaibol Q bezeichnet eine Verbindung der allgemeinen Formel II, in der X Pro bedeutet.

AcPhe steht für N-Acetylphenylalanin, Aib für α-Aminoisobuttersäure, Ala für Alanin, Iva für Isovalin, Hyp für Hydroxyprolin, Phe-ol für Phenylalaninol, Phe für Phenylalanin, Gly für Glycin, Leu für Leucin, Gln für Glutamin, Pro für Prolin, Ile für Isoleucin, Thr für Threonin und Ser für Serin.

Die erfindungsgemäßen Cephaibole werden durch Acremonium tubakii , bevorzugt durch Acremonium tubakii FH 1685 DSM 12774 produziert.
Acremonium tubakii FH 1685 DSM 12774 besitzt ein beigerotes Mycel und ist durch die für Acremonium-Arten charakteristischen Conidiophoren gekennzeichnet.
Ein Isolat wurde bei der Deutschen Sammlung von Mikroorganismen und Zellkulturen GmbH, Mascheroder Weg 1B, D 38124 Braunschweig, Deutschland, nach den Regeln des Budapester Vertrages am 31. März 1999 unter der folgenden Nummer hinterlegt: Acremonium tubakii FH 1685 DSM 12774.

In einer Nährlösung (auch als Kulturmedium bezeichnet), die eine Kohlenstoffquelle und eine Stickstoffquelle sowie die üblichen anorganischen Salze enthält,. produziert Acremonium tubakii FH 1685 DSM 12774 eine oder mehrere der erfindungsgemäßen Verbindungen der Formel I oder II.

Anstelle des Stammes Acremonium tubakii FH 1685 DSM 12774 können auch dessen Mutanten und Varianten eingesetzt werden, die ein oder mehrere Verbindungen der erfindungsgemäßen Cephaibole synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) oder unter Anwendung gentechnischer Methoden erzeugt werden.

Das Screening nach Mutanten und Varianten, die ein oder mehrere der erfindungsgemäßen Cephaibole synthetisieren, erfolgt nach folgendem Schema:
- Abtrennung des Mycels nach der Fermentation;
- Extraktion der Mycels mit einem organische Lösungsmittel;
- Extraktion der Cephaibole aus dem Kulturfiltrat mit Festphasen oder mit Wasser nicht mischbaren organischen Lösungsmittel:
- Analytik mittels HPLC, DC oder durch Testen der biologischen Wirksamkeit.

Die im folgenden beschriebenen Fermentationsbedingungen gelten für Acremonium tubakii, das hinterlegte Isolat Acremonium tubakii FH 1685 DSM 12774 sowie Mutanten und Varianten von diesen.

Das erfindungsgemäße Verfahren kann für die Fermentation im Labormaßstab (Milliliter- bis Literbereich) und im industriellen Maßstab (Kubikmetermaßstab) eingesetzt werden. Alle Prozentangaben beziehen sich, wenn nichts anderes angegeben ist, auf das Gewicht. Mischungsverhältnisse bei Flüssigkeiten beziehen sich auf das Volumen, wenn keine anderen Angaben gemacht werden.

In einem Kulturmedium, das eine Kohlenstoffquelle und eine Stickstoffquelle sowie die üblichen anorganischen Salze enthält, produziert Acremonium tubakii FH 1685 DSM 12774 die erfindungsgemäßen Verbindungen der Formel I oder II.

Als bevorzugte Kohlenstoffquellen für die aerobe Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Cephaibole verläuft besonders gut in einem Kulturmedium, das etwa 0,1 bis 5 %, bevorzugt 0,3 bis 2 % Hefeextrakt und 0,2 bis 5 %, bevorzugt 0,5 bis 3 % Saccharose und 0,1 bis 10 g/l, bevorzugt 0,2 bis 1,0 g/l Magnesiumsulfat und 0,05 bis 1,0 g/l, bevorzugt 0,1 bis 0,5 g/l Kalium- oder Natriumdihydrogen-phosphat und 0,05 bis 1,0 g/l, bevorzugt 0,1bis 1,0 g/l Natriumnitrat und 0,01 bis 0,1 g/l bevorzugt 0,02 bis 0,1 g/l Kaliumchlorid und 0,01 bis 100 µM, bevorzugt 5 bis 20 µM Eisen-Sulfat sowie Spuren von Zinksulfat und Kupfersulfat enthält. Die Angaben in Prozent sind jeweils bezogen auf das Gewicht des gesamten Kulturmediums.

In dem Kulturmedium bildet Acremonium tubakii FH 1685 DSM 12774 ein Gemisch aus Cephaibolen. Je nach Zusammensetzung des Kulturmediums kann der mengenmäßige Anteil eines oder mehrerer der erfindungsgemäßen Cephaibole variieren. Außerdem kann durch die Medienzusammensetzung die Synthese einzelner Cephaibole gesteuert werden, so daß eines oder auch mehrere der Cephaibole gar nicht bzw. in einer Menge unterhalb der Nachweisgrenze vom Mikroorganismus hergestellt werden.

Vorzugsweise besteht das Gemisch aus 8 unterschiedlichen, nachweisbaren Cephaibolen (Cephaibole A, A1, B, C, D, E sowie P und Q). Bevorzugt werden die Cephaibole A bis E gebildet.

Neben den Cephaibolen A1 und A bis E (Verbindungen der Formel I , die als charakteristisches Element am C-Terminus Phenylalaninol (Phe-o116) tragen), werden in dem Kulturmedium des Acremonium tubakii FH 1685 DSM 12774 auch Verbindungen der Formel I gebildet, die als C-terminale Gruppe den Aldehyd Phenylalaninal (Phe-al16) an Stelle von Phe-ol16 tragen. Diese stellen in der Regel Minoritätenprodukte zu den Cephaibolen A-E dar und werden zusammen mit diesen aus dem Kulturmedium gewonnen.
Die Kultivierung des Mikroorganismus erfolgt aerob, also beispielsweise submers unter Schütteln oder Rühren in Schüttelkolben oder Fermentem, gegebenenfalls unter Einführen von Luft oder Sauerstoff. Sie kann in einem Temperaturbereich von etwa 18 bis 35°C, vorzugsweise bei etwa 20 bis 30°C, insbesondere bei 22 bis 28°C durchgeführt werden. Der pH-Bereich sollte zwischen 6 und 8 liegen, vorzugsweise zwischen 6,5 und 7,5. Man kultiviert den Mikroorganismus unter diesen Bedingungen im allgemeinen über einen Zeitraum von 24 bis 300 Stunden, vorzugsweise 36 bis 140 Stunden.

Vorteilhaft kultiviert man in mehreren Stufen, d. h. man stellt zunächst eine oder mehrere Vorkulturen in einem flüssigen Kulturmedium her, die dann in das eigentliche Produktionsmedium, die Hauptkultur, beispielsweise im Volumenverhältnis 1:10, überimpft werden. Die Vorkultur erhält man, z.B., indem man ein Mycel in eine Nährlösung überimpft und etwa 36 bis 120 Stunden, bevorzugt 48 bis 72 Stunden, wachsen läßt. Das Mycel kann beispielsweise erhalten werden, indem man den Stamm etwa 3 bis 40 Tage, bevorzugt 4 bis 10 Tage, auf einem festen oder flüssigen Nährboden, beispielsweise Malz-Hefe-Agar oder Potatodextrose-Agar (Standardmedium für Schimmelpilze z.B. von Fa. Difco) wachsen läßt.

Der Fermentationsverlauf kann anhand des pH-Wertes der Kulturen oder des Mycelvolumens sowie durch chromatographische Methoden, wie z.B. Dünnschichtchromatographie oder High Pressure Liquid Chromatography oder Ausprüfen der biologischen Aktivität überwacht werden. Die erfindungsgemäßen Cephaibole sind sowohl im Mycel als auch im Kulturfiltrat enthalten, der größte Teil befindet sich jedoch im Mycel.

Das im folgenden beschriebene Isolierungsverfahren dient zur Aufreinigung der erfindungsgemäßen Cephaibole, vorzugsweise zur Aufreinigung der Cephaibole A, A1, B, C und D.

Die Isolierung bzw. Aufreinigung der erfindungsgemäßen Cephaibole aus dem Kulturmedium erfolgt nach bekannten Methoden unter Berücksichtigung der chemischen, physikalischen und biologischen Eigenschaften der Naturstoffe. Zum Testen der Peptidwirkstoff-Konzentration im Kulturmedium oder in den einzelnen Isolierungsstufen kann die Dünnschichtchromatographie, beispielsweise an Kieselgel mit Isopropanol/25%igem NH₃ als Laufmittel oder HPLC verwendet werden. Die Detektion bei der dünnschichtchromatographischen Auftrennung kann beispielsweise durch Färbereagentien wie Anisaldehyd/Schwefelsäure erfolgen, wobei die Menge der gebildeten Substanz zweckmäßig mit einer Eichlösung verglichen wird. Die Nebenprodukte der Formel I, in denen R ein Phenylalaninal-Rest bedeutet, können gegebenenfalls von dem jeweiliegen Cephaibol A-E durch gängige, dem Fachmann bekannten Reinigungsmethoden abgetrennt werden, beispielsweise durch Chromatographie oder durch Umkristallisation.

Zur Isolierung der erfindungsgemäßen Cephaibole wird das Mycel zunächst von dem Kulturmedium nach den üblichen Verfahren abgetrennt und anschließend werden die Cephaibole von der Zellmasse mit einem gegebenenfalls mit Wasser mischbaren organischen Lösungsmittel extrahiert. Die organische Lösungsmittelphase enthält die erfindungsgemäßen Cephaibole, sie werden gegebenenfalls im Vakuum konzentriert und wie unten beschrieben weiter aufgereinigt.

Das Kulturfiltrat wird gegebenenfalls mit dem Konzentrat des Mycel-Extraktes vereinigt und mit einem geeigneten, mit Wasser nicht mischbarem organischen Lösungsmittel, zum Beispiel mit n-Butanol, extrahiert. Die anschließend abgetrennte organische Phase wird ggf. im Vakuum konzentriert. Man kann das Konzentrat zum entfetten des Wertproduktes mit einem unpolaren Lösungsmittel, in dem die erfindungsgemäßen Cephaibole sehr wenig löslich sind, wie zum Beispiel mit Hexan, Petroleum-ether, Diethylether verdünnen. Hierbei präzipitieren die Cephaibole, die lipophilen Verunreinigungen bleiben gelöst und werden durch übliche Fest/flüssig-Phasentrennungen entfernt.
Der Niederschlag, der die gesamten Cephaibole enthält, wird in 1/30 des ursprünglichen Volumens Wasser/Methanol gelöst. Der Niederschlag geht dabei vollständig in Lösung und wird lyophilisiert. Das Lyophilisat, in der Folge als Rohprodukt bezeichnet, enthält 5 bis 50 % Cephaibole und wird für die weitere Isolierung eingesetzt.

Die weitere Aufreinigung eines oder mehrerer der erfindungsgemäßen Cephaibole erfolgt durch Chromatographie an geeigneten Materialien, vorzugsweise z.B. an Molekularsieben, an Kieselgel, Aluminiumoxid, an Ionenaustauschern oder an Adsorberharzen bzw. an Umkehr-Phasen (reversed phase, RP). Mit Hilfe dieser Chromatographie werden die Cephaibole getrennt. Die Chromatographie der Cephaibole erfolgt mit gepufferten wässrigen Lösungen oder Gemischen von wässrigen und organischen Lösungen.

Unter Gemischen von wässrigen und organischen Lösungen versteht man alle mit Wasser mischbaren organischen Lösemittel, vorzugsweise Methanol, Acetonitril, in einer Konzentration von 10 bis 80 % Lösemittel, vorzugsweise 40 bis 60 % Lösemittel oder auch alle gepufferten wässrigen Lösungen, die mit organischen Lösemitteln mischbar sind.

Die Trennung der Cephaibole aufgrund ihrer unterschiedlichen Polarität erfolgt mit Hilfe der Reversed Phase Chromatographie, beispielsweise an MCl® (Adsorberharz von Mitsubishi, Japan) oder Amberlite XAD® (TOSOHAAS), an weiteren hydrophoben Materialien, wie zum Beispiel an RP-8- oder RP-18-Phasen.
Außerdem kann die Trennung mit Hilfe der Normalphasen-Chromatographie, zum Beispiel an Kieselgel, Aluminiumoxyd und ähnlichen erfolgen.

Die Chromatographie der Cephaibole erfolgt mit gepufferten oder angesäuerten wäßrigen Lösungen oder Gemischen von wäßrigen Lösungen mit Alkoholen oder anderen, mit Wasser mischbaren organischen Lösemitteln. Als organische Lösemittel werden vorzugsweise Propanol und Acetonitril verwendet.

Unter gepufferten oder angesäuerten wäßrigen Lösungen versteht man z.B. Wasser, Phosphatpuffer, Ammoniumacetat, Citratpuffer in einer Konzentration von 0,1 mM bis 0,5 M sowie Ameisensäure, Essigsäure, Trifluoressigsäure oder allen handelsüblichen, dem Fachmann bekannten Säuren, vorzugsweise in einer Konzentration von 0,01 bis 1 %. Besonders bevorzugt wird 0,1 %.

Chromatographiert wird mit einem Gradienten, der mit 100 % Wasser beginnt und mit 100 % Lösemittel endet, vorzugsweise wird ein linearer Gradient von 30 bis 60 % Propanol oder Acetonitril gefahren.

Alternativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad) oder Fractogel TSK HW 40® (Fa. Merck, Deutschland, Toso Haas, USA) oder Sephadex ® (Pharmacia, Schweden).

Die Reihenfolge der vorgenannten Chromatographien ist umkehrbar.

Ein weiterer, sehr wirksamer Reinigungsschritt für Cephaibole ist die Kristallisation. Die Cephaibole kristallisieren aus Lösungen in organischen Lösungsmitteln und aus Mischungen von Wasser mit organischen Lösungsmitteln leicht aus. Die Kristallisation wird in an sich bekannter Weise, zum Beispiel durch Konzentrieren oder Abkühlen gesättigter Peptidwirkstoff-Lösungen, durchgeführt.

Die erfindungsgemäßen Cephaibole sind im festen Zustand und in Lösungen im pH-Bereich zwischen 3 und 8, insbesondere 5 und 7, stabil und lassen sich damit in übliche galenische Zubereitungen einarbeiten.

Unter physiologisch verträglichen Salzen von Verbindungen der Formel I oder II versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calciumund Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die vorliegende Erfindung umfaßt alle stereoisomeren Formen der Verbindungen der Formel I oder II. In den Verbindungen der Formel II enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung.

Die vorliegende Erfindung umfaßt auch offensichtlich chemische Äquivalente der Verbindungen der Formel I oder II. Solche sind z.B. Ester, Ether, Additionssalze, Komplexe, oder auch Partialhydrolyseprodukte.

Aufgrund ihrer wertvollen pharmakologischen Eigenschaften eignen sich die erfindungsgemäßen Cephaibole zur Anwendung als Arzneimittel in der Humanund/oder Tiermedizin. Die erfindungsgemäßen Substanzen besitzen pharmakologische Wirksamkeit, insbesondere als Antibiotikum gegen Parasiten, besonders bevorzugt gegen Endo- und/oder Ektoparasiten.

Die vorliegende Erfindung betrifft somit ferner die Verwendung der erfindungsgemäßen Verbindungen der Formel I oder II als Human- oder Tierpharmaka, insbesondere als Chemotherapeutika gegen human- und/oder tierpathogene Endo- und/oder Ektoparasiten. Der Wirkmechanismus dieser Peptidwirkstoffe ist unbekannt, jedoch konnte ein signifikanter, letaler Effekt gegen Helminthen und Ektoparasiten nachgewiesen werden.

Die erfindungsgemäßen Cephaibole eignen sich beispielsweise zur Bekämpfung tier- und humanpathogene Trematoden (Fasciola hepatica, Fasciolopsis buski, Fasciola gigantica, Fascioloides magna, Dicrocoelium dendriticum, Opistorchis felineus, Clonorchis sinensis, Paragonimus westermanni, Paragonimus kellikotti, Schistosoma haematobium, Schistosoma japonicum, Schistosoma mansoni) und tier- und humanpathogene Nematoden, die zur Familie der Trichuridae, Trichinellidae, Strongyloididae, Ancylostomatidae, Strongylidae, Trichostrongylidae, Metastrongylidae, Oesophagostomatidae, Dictyocaulidae, Protostrongylidae, Angiostrongylidae, Oxyuridae, Ascaridae, Toxocaridae, Dracunculidae, Habronematidae und Filariidae zählen; darüberhinaus sind die erfindungsgemäßen Cephaibole zur Bekämfung von tier- und humanpathogene Ektoparasiten die zur Klasse der Arachnida (Familie: Argasidae, Ixodidae, Dermanyssidae; Demodicidae, Sarcoptidae, Psoroptidae, Varroidae) und zur Klasse der Insecta zählen, die die Ordnung der Phthiraptera (Anoplura, Mallophaga), Diptera und Siphonaptera umfassen, geeignet.

Neben der antibakteriellen Wirkung besitzen die erfindungsgemäßen Verbindungen antimykotische, d. h. pilzhemmende Eigenschaften einschließlich der pflanzenpathogenen Pilze.

Bei Parasiten, die Resistenzen gegen herkömmliche Mittel ausgebildet haben, besitzen nur neue Agentien eine therapeutisch ausreichende Wirkung. Die erfindungsgemäßen Cephaibole der Formel I oder II weisen somit potentiell eine hervorragende Wirkung auch gegen diese Problem-Organismen auf.

Aufgrund der antibakteriellen, antimykotischen und antiprotozoen Aktivität besitzen die erfindungsgemäßen Cephaibole wachstumsfördernde Wirkungen, die in der Tierproduktion nutzbringend angewandt werden können. Die verbesserte Futterverwertung kann bei Nutztieren wie beispielsweise Rindern, Schafen, Ziegen, Schweinen, Pferden oder Kaninchen genützt werden. Hierzu verwendet man vorzugsweise Dosierungen von 0,05 - 50 mg/kg/Tag. Die Cephaibole bewirken dabei auch eine Abnahme der Methangas Produktion und eine Verbesserung der Futterverwertung.

Die Erfindung betrifft auch pharmazeutische Zubereitungen, die eine oder mehrere der erfindungsgemäßen Cephaibole enthalten. Bevorzugt ist die Verwendung in Mischung mit geeigneten Hilfsstoffen oder Trägermaterial. Als Trägermaterial können bei Tierarzneimitteln die üblichen Futtermittelmischungen bzw. beim Menschen alle pharmakologisch verträglichen Trägermaterialien und/oder Hilfsstoffe verwendet werden.

Die Erfindung betrifft auch ein Verfahren zur Herstellung eines erfindungsgemäßen Arzneimittels, das dadurch gekennzeichnet ist, daß man mindestens eine der erfindungsgemäßen Verbindungen mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Die erfindungsgemäßen Arzneimittel werden im allgemeinen oral, lokal oder parenteral verabreicht, aber auch eine rektale Anwendung ist prinzipiell möglich.

Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Cephaibole enthält. Bei festen Dosierungseinheiten wie Tabletten, Kapseln und Suppositorien kann diese Dosis bis zu etwa 2000 mg, bevorzugt jedoch etwa 1 bis 1000 mg, und bei Injektionslösungen in Ampullenform bis zu etwa 1000 mg, vorzugsweise aber etwa 10 bis 300 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man ein oder mehrere Verbindungen der erfindungsgemäßen Cephaibole mit üblichen Träger- sowie gegebenenfalls Zusatz- und/oder Hilfstoffe in die bzw. eine geeignete Darreichungsform bringt.

Grundsätzlich sind bei der Behandlung von Helminthen und Ektoparasiten therapeutische, meta- und prophylaktische Maßnahmen zu unterscheiden; diese verschiedenen Behandlungsmethoden erfordern spezielle, galenische Formulierungen. Solche Formulierungen gewährleisten z.B. die kontinuierliche Gabe von subtherapeutischen bis therapeutischen Dosen von Anthelminthika bzw. von Ektoparasitiziden, die nach ihrem Freisetzungsmodus in "Sustained-" und "Pulse-Release Boli" unterschieden werden. Erstere unterscheidet man weiter in "Slow-Release Boli", das sind solche mit abnehmender Freisetzungsrate und "Continous-Release Boli", das sind solche mit gleichbleibendem Freisetzungsmodus. "Pulse-Release Boli" setzen hingegen den gesamten Wirkstoff innerhalb weniger Stunden bis Tage frei. Neben der Release-Technologie, die auch die Mikroverkapselung von Wirkstoffen beinhaltet und die sich zunehmender Beliebtheit in der Veterinärmedizin erfreut, sind sogenannte "spot on" bzw. "pour on" Formulierungen für die äußere Anwendung am Tier üblich; die Verabreichung von Tabletten, Pasten oder Injektionslösungen sowie die Anwendung von Halsbändern, die Medikamente gegen z.B. Ektoparasiten enthalten, ist Stand der Technik.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### Beispiel 1

### Herstellung einer Glycerinkultur von Acremonium tubakii FH 1685 DSM 12774

100 ml Nährlösung (Malzextrakt 2,0%, Hefeextract 0,2 %, Glucose 1,0 %, (NH₄)₂HPO₄ 0,05 %, pH 6,0) in einem sterilen 300 ml Erlenmeyerkolben werden mit dem Stamm Acremonium tubakii FH 1685 DSM 12774 beimpft und 7 Tage bei 25° C und 140 UpM auf einer rotierenden Schüttelmaschine inkubiert. 1,5 ml dieser Kultur werden anschließend mit 2,5 ml 80 %igem Glycerin verdünnt und bei -20°C gelagert.

### Beispiel 2

### Herstellung einer Kultur bzw. einer Vorkultur im Erlenmeyerkolben von Acremonium tubakii FH 1685 DSM 12774

Ein steriler 300 ml Erlenmeyerkolben mit 100 ml der folgende Nährlösung 30 g/l Saccharose, 5 g/l Hefeextrakt, 1 g/l K₂HPO₄, 3 g/l NaNO₃, 0,5 g/l MgSO₄ x 7 H₂O, 0,01 g/l FeSO₄ x 7 H₂O, 0,5 g/l KCl und 1,0 ml Spurenelementlösung (0,2 g/l ZnSO₄ x 7 H₂O und 0,7 g/l CuSO₄ x 5 H₂O) wird mit einer auf einem Schrägröhrchen (gleiche Nährlösung, jedoch mit 2 % Agar) gewachsenen Kultur oder mit 1 ml einer Glycerinkultur (s. Beispiel 1) beimpft und auf einer Schüttelmaschine bei 180 UpM und 30°C inkubiert. Die maximale Produktion einer oder mehrerer Verbindungen der erfindungsgemäßen Cephaibole ist nach ca. 120 Stunden erreicht. Zum Animpfen von 10 und 200 I Fermentern genügt eine 48 bis 96 Std. alte Submerskultur (Animpfmenge ca. 10 %) aus der gleichen Nährlösung.

### Beispiel 3

### Herstellung der Cephaibole

Ein 30 I Fermenter wird unter folgenden Bedingungen betrieben:
- Nährmedium:: 30 g/l Saccharose
5 g/l Hefeextrakt
3 g/l NaNO₃
0,5 g/l KCI
0,5 g/l MgSO₄
0,1 g/l K₂HPO₄
10 µM FeCl₃ x 6H₂O
1 ml Spurenelementlösung
pH 6,5 (Vor der Sterilisation)
- Spurenelementtösung:: 2 g/l ZnSO₄ x 7H₂O sowie 0,7 g/l CuSO₄ x 5H₂O.
- Inkubationszeit:: 50 Stunden
- Inkubationstemperatur:: 25° C
- Rührergeschwindigkeit:: 300 UpM
- Belüftung:: 15 l Min⁻¹

Durch wiederholte Zugabe von ethanolischer Polyollösung kann die Schaumbildung unterdrückt werden. Das Produktionsmaximum wird nach ca. 96 bis 120 Stunden erreicht.

### Beispiel 4

### Isolierung des Cephaibol-Gemisches aus der Kulturlösung des Acremonium tubakii FH 1685 DSM 12774

Nach Beendigung der Fermentation von Acremonium tubakii FH 1685 DSM 12774 wird die Kulturbrühe von drei Fermentem, gewonnen nach Beispiel 3, (90 Liter) unter Zusatz von ca. 2 % Filterhilfsmittel (z.B. Celite®) filtriert und die Zellmasse (6 Liter) mit 20 Liter Methanol extrahiert. Die Pepdtidwirkstoff-haltige, methanolische Lösung wird durch Filtration vom Mycel befreit und im Vakuum konzentriert. Das Konzentrat wird gemeinsam mit dem Kulturfiltrat (83 Liter) auf eine vorbereitete, 4 Liter MCI GEL, CHP20P-Säule aufgetragen. Eluiert wird mit einem Gradienten von 0.1 % Trifluoressigsäure in Wasser nach 0.1% Trifluoressig-säure in Propanol-2. Der Säulendurchfluß (12 Liter pro Stunde) wird fraktioniert aufgefangen (je 2.5 Liter) und die die Peptidwirkstoffe enthaltenden Fraktionen (21 bis 24) zusammengefaßt. Konzentrieren im Vakuum und Gefriertrocknung ergeben 4 g eines hellbraunen Pulvers.

### Beispiel 5

### Anreicherung der Cephaibol-Komponenten durch Gel-Chromatographie.

4 g des nach Beispiel 4 gewonnenen Produktes werden auf eine 3,9 Liter fassende mit Fractogel® TSK MW-40 s gefüllte Säule (Breite x Höhe = 10 cm x 50 cm) aufgetragen. Das Laufmittel Methanol wird mit einer Flußrate von 50 ml pro Minute über die Säule gepumpt und der Säulenausfluß fraktioniert (65 ml) aufgefangen.Hauptsächlich in den Fraktionen 28 bis 33 befinden sich die Cephaibole. Sie werden zusammengefaßt und im Vakuum vom Methanol befreit. Sie ergeben 1.3 g Peptidwirkstoffgemisch.

### Beispiel 6

### Trennung der Cephaibol-Komponenten auf Reverse Phase RP-18.

Es wird eine 500 ml fassende präparative HPLC-Säule (5,1 cm (ID) x 25 cm H) mit ®Nucleosil 100-7 C18 HD, gefüllt und die 1,3 g des Peptidwirkstoffgemisches, gewonnen nach Beispiel 5, aufgetragen. Eluiert wird mit 40 % Acetonitril in 0,1% wässriger Trifluoressigsäure-Lösung. Der Säuiendurchfiuß beträgt 50 ml / Minute, es werden Fraktionen von je 125 ml Inhalt gesammelt. In den Fraktion 46 befindet sich das Cephaibol D, in Fraktionen 49 und 50 Cephaibol C, in Fraktion 51 Cephaibol E, in den Fraktionen 60 bis 64 Cephaibol A und in Fraktion 66 sowie 67 Cephaibol B sowie A1. Fraktion 68 beinhaltet ein Gemisch von Cephaibolen. Nach dem Konzentrieren im Vakuum und Gefriertrocknung werden folgende Mengen ausgewogen:
Cephaibol A: 520 mg, ESI+ MS: 1671 Da ( M+H )⁺,
Cephaibol A1: 4 mg, ESI+ MS: 1685 DA (M+H)⁺
Cephaibol B: 38 mg, ESI+ MS: 1685 Da ( M+H )⁺,
Cephaibol C:195 mg, ESI+ MS: 1657 Da ( M+H )⁺,
Cephaibol D: 16 mg, ESI+ MS: 1643 Da ( M+H )⁺,
Cephaibol E: 76 mg, ESI+ MS: 1657 Da ( M+H )⁺.

### Beispiel 7

### Isolierung der Cephaibole P und Q.

Die Fraktion 68, gewonnen nach Beispiel 6, wird nach Gefriertrocknung (4,1 mg), gelöst in 20% Acetonitril in Wasser, auf eine 250/10 Nucleosil C18 300-7® - Säule aufgetragen. Die Elution erfolgt mit 0,005% Ammoniumacetat-Puffer in 40% Acetonitril. Neben wenig Cephaibol B werden nach Trocknung 1 mg Cephaibol P und 1 mg Cephaibol Q gewonnen.

Cephaibol P: ESI+ MS wird ein Molekulargewicht von 1873 ( M+H )⁺ gemessen, Cephaibol Q: ESI+ MS wird ein Molekulargewicht von 1857 ( M+H )⁺ gemessen.

### Beispiel 8

### HPLC System zum Nachweis der Cephaibole.

Das unten beschriebene System erlaubt die Trennung und Quantifizierung der Cephaibole im Rohgemisch bzw. im Kulturfiltrat; die Retentionszeiten liegen zwischen 7,0 Minuten (Cephaibol D) und 18,8 Minuten (Cephaibol A1).
- Laufmittel:: 0,1 % Trifluoressigsäure in 40 % Acetonitril.
- Säule:: Nucleosil 100C₁₈AB 250/4, Macherey-Nagel.
- Fluß:: 1,0 ml/Min
- Detektion:: Ultravioletlicht-Absoption bei 210 nm.

Unter den angegebenen Bedingungen haben die Cephaibole folgende Retentionszeiten:
Cephaibol A: 12,1 Minuten,
Cephaibol A1: 18,8 Minuten,
Cephaibol B: 16,0 Minuten,
Cephaibol C: 9,0 Minuten,
Cephaibol D: 7,1 Minuten,
Cephaibol E : 7,3 Minuten,
Cephaibol P : 17,3 Minuten,
Cephaibol Q : 17,9 Minuten.

### Beispiel 9

### Charakterisierung des Cephaibol A.

10 µg Cephaibol A werden in konstant-siedender Salzsäure hydrolysiert und auf einem Aminosäuren-Analysator untersucht. Es werden folgende übliche Aminosäuren gefunden:

| | |
|---|---|
| Hydroxyprolin | 11,5 nMol |
| Glutaminsäure | 5,7 nMol |
| Prolin | 5,5 nMol |
| Glycin | 5,5 nMol |
| Leucin | 5,6 nMol |
| Phenylalanin | 5,5 nMol |

Die Gehalte der ungewöhnlichen Aminosäuren Amino-iso-Buttersäure - und Iso-Valin waren nicht auswertbar.

Massenspektometrische Untersuchungen mit einem Finnigan MAT LCQ Ionenfallen Massenspektrometer mit Elektronen-spray ionisation (ESI +) ergeben folgende Daten:

Das ESI Massenspektrum zeigt ein intensives MH⁺ bei m/z 1670.7 und ein [M+Na]⁺ bei m/z 1692.7 entsprechend einem monoisotopischen Molekulargewicht of 1669.7, in guter Übereinstimmung mit der berechneten Masse (für C₈₂H₁₂₇N₁₇O₂₀, monoisotopisch) von 1669.9 Da. Das MS/MS-Spektrum zeigt eine Fragmentierung, die in der Tabelle 9 in Fettdruck aufgelistet ist. Die kursiv geschriebenen Fragmente sind berechnete, aber nicht beobachtete Fragmente.

**Tabelle 9:**

| MS/MS-Fragmentierung des Cephaibol A | | | | | |
|---|---|---|---|---|---|
| B₁ | **190,1** | B₁ | *212,1* | A₁ | *184,1* |
| B₂ | **275,1** | B₂ | *297,1* | A₂ | *269,1* |
| B₃ | **360,2** | B₃ | 382,2 | A₃ | 354,2 |
| B₄ | **445,2** | B₄ | *467,2* | A₄ | *439,2* |
| B₅ | **530,3** | B₅ | *552,3* | A₅ | *524,3* |
| B₆ | **587,3** | B₆ | *609,3* | A₆ | *581,3* |
| B₇ | **700,4** | B₇ | **722,4** | A₇ | **694,4** |
| B₈ | **799,5** | B₈ | **821,5** | A₈ | **793,5** |
| B₉ | **884,5** | B₉ | **906,5** | A₉ | **878,5** |
| B₁₀ | **997,6** | B₁₀ | **1019,6** | A₁₀ | *991,6* |
| B₁₁ | *1125,6* | B₁₁ | **1147,6** | A₁₁ | **1119,6** |
| B₁₂ | **1224,7** | B₁₂ | **1246,7** | A₁₂ | **1218,7** |
| B₁₃ | **1337,7** | B₁₃ | **1359,7** | A₁₃ | **1331,7** |
| B₁₄ | **1422,8** | B₁₄ | **1444,8** | A₁₄ | **1416,8** |
| B₁₅ | **1519,9** | B₁₅ | *1541,9* | A₁₅ | *1513,9* |

In der ersten Spalte ist die protonierte B - und in der zweiten Spalte die (M+Natrium)⁺-B - Fragmentierung aufgeführt. Die dritte Spalte zeigt die A-Fragmentreihe in der Na-Form.

Die physikalisch-chemischen sowie spektroskopischen Eigenschaften der erfindungsgemäßen Cephaibole lassen sich wie folgt zusammenfassen:

### Cephaibol A:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₂H₁₂₇N₁₇O₂₀,
Strukturformel: 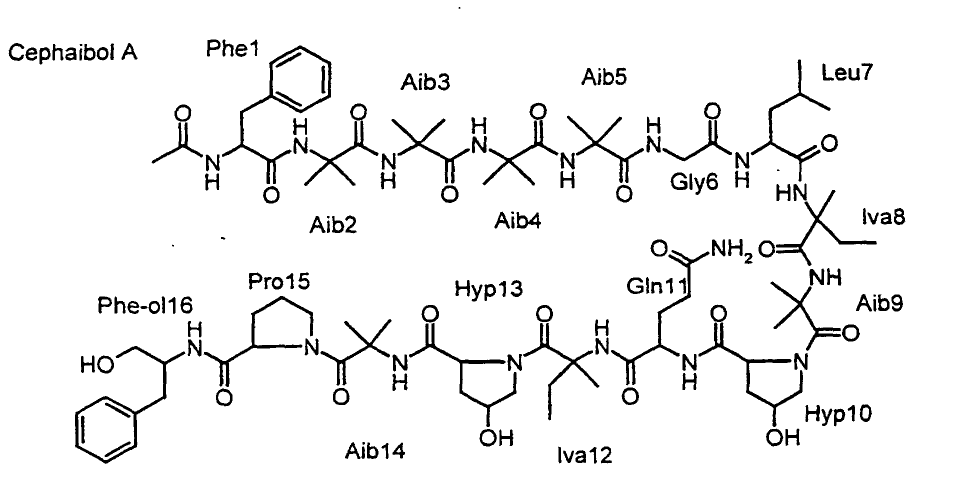 Molekulargewicht: 1671 Da,
UV-Absorption (λₘₐₓ): 258 nm, log ε: 2.62
NMR-Daten: siehe Tabelle 1

### Cephaibol A1:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₃H₁₂₉N₁₇O₂₀, 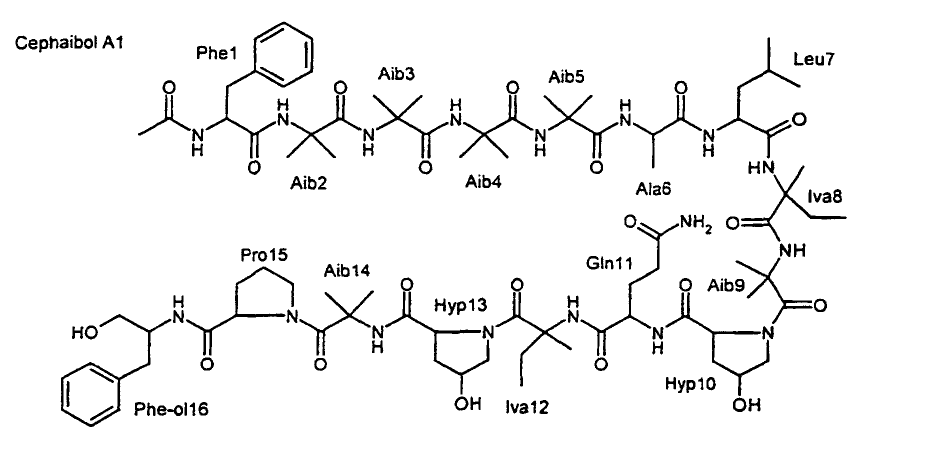 Molekulargewicht: 1685 Da,
UV-Daten (λₘₐₓ): 258 nm, log ε: 2,62
NMR-Daten: siehe Tabelle 1a

### Cephaibol B:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₃H₁₂₉N₁₇O₂₀,
Strukturformel: 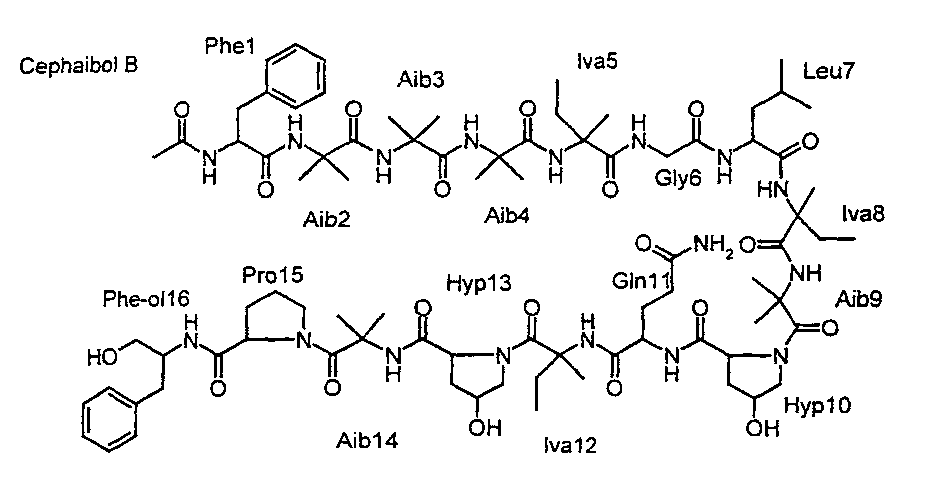 Molekulargewicht: 1685 Da,
UV-Absorption (λₘₐₓ): 258 nm, log ε: 2.62
NMR-Daten: siehe Tabelle 2.

### Cephaibol C:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₁H₁₂₅N₁₇O₂₀,
Strukturformel: 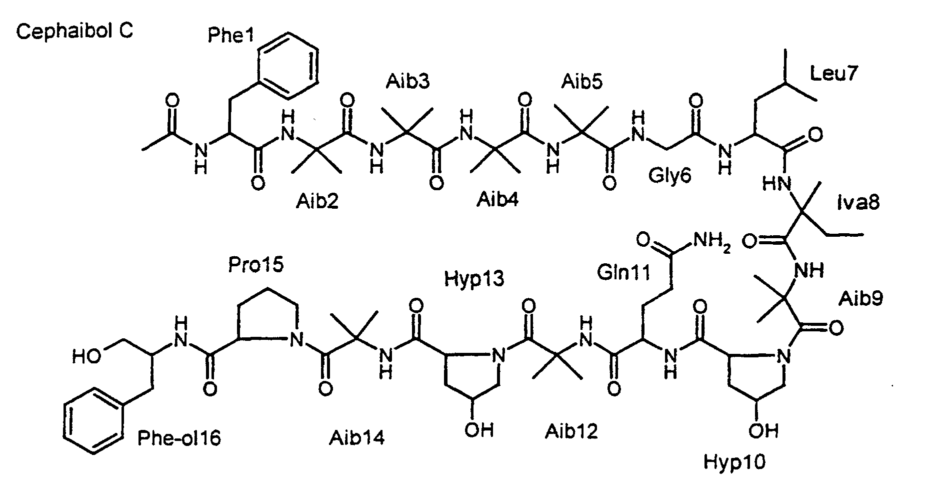 Molekulargewicht: 1657 Da,
UV-Absorption (λₘₐₓ): 258 nm, log ε: 2.62
NMR-Daten: siehe Tabelle 3.

### Cephaibol D:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₀H₁₂₃N₁₇O₂₀,
Strukturformel: 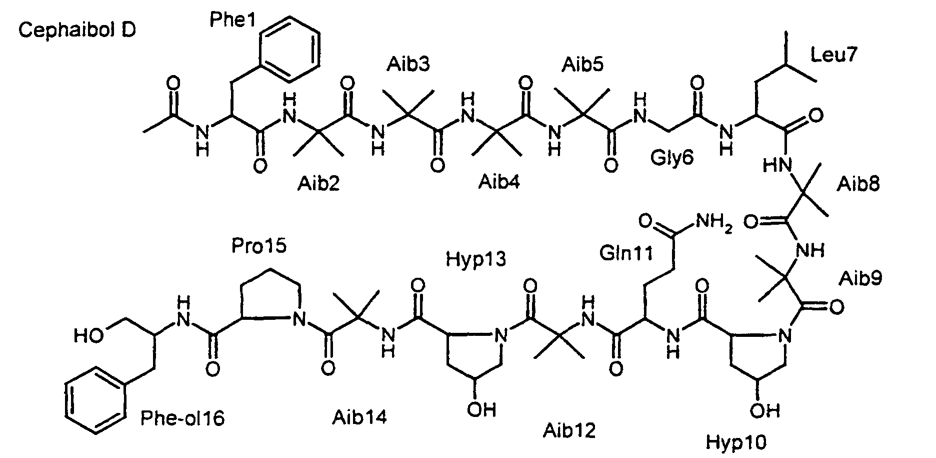 Molekulargewicht: 1643 Da,
UV-Absorption (λₘₐₓ): 258 nm, log ε: 2.62
NMR-Daten: siehe Tabelle 4.

### Cephaibol E:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₁H₁₂₅N₁₇O₂₀,
Strukturformel: 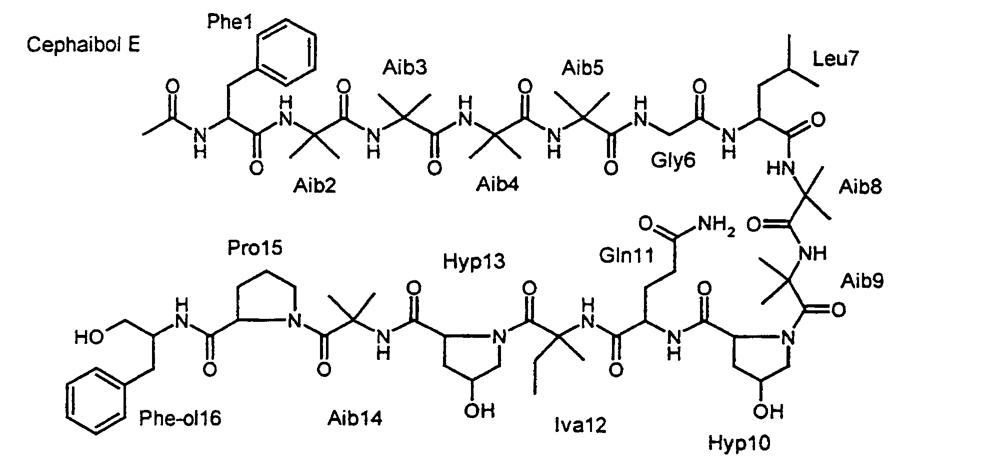 Molekulargewicht: 1657 Da,
UV-Absorption (λₘₐₓ): 258 nm, log ε: 2.62
NMR-Daten: siehe Tabelle 5.

### Cephaibol P:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₉H₁₃₇N₁₉O₂₅,
Strukturformel: 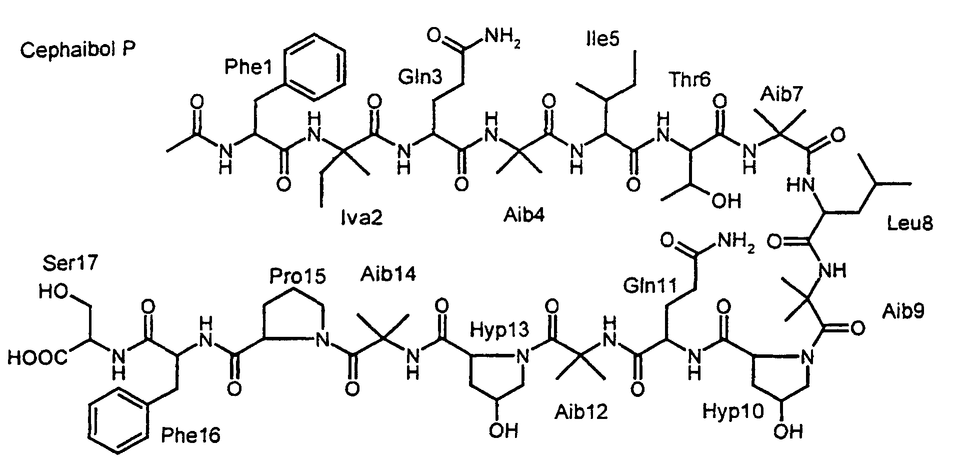 Molekulargewicht: 1873 Da,
UV-Absorption (λₘₐₓ): 258 nm, log ε: 2.62
NMR-Daten: siehe Tabelle 6.

### Cephaibol Q:

Aussehen: in polaren organischen Lösungsmitteln lösliche, jedoch in Wasser nur wenig lösliche, farblose Substanz. Sie ist stabil im neutralen, mild-saurem und mildalkalischen Milieu.
Summenformel: C₈₉H₁₃₇N₁₉O₂₄,
Strukturformel: 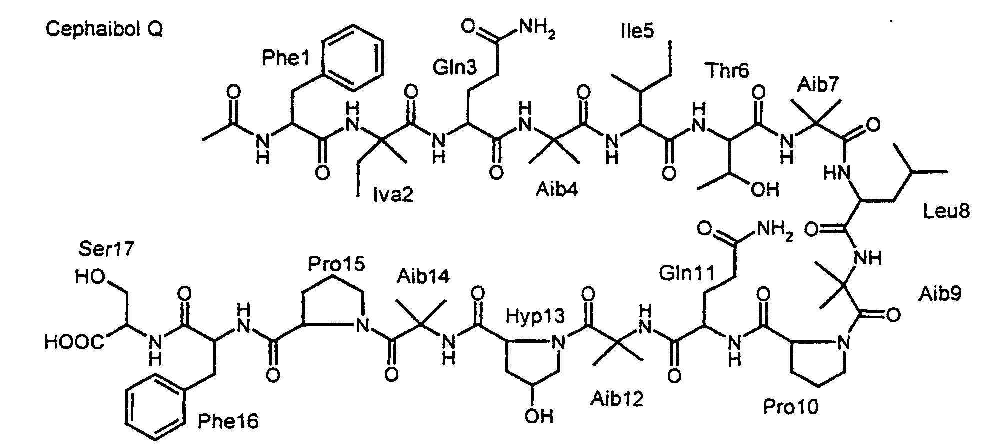 Molekulargewicht: 1857,197 Da,
UV-Absorption (λₘₐₓ): 258 nm, log ε: 2.62
NMR-Daten: siehe Tabelle7.

**Tabelle 1:**

| NMR-Daten (chemische Verschiebungen) des Cephaibols A in DMSO bei 300° K. | | |
|---|---|---|
| Proton/Kohlenstoff | ¹H | ¹³C |
| | | |
| Ac-Me | 1.83 | 22.29 |
| Ac-C' | - | 170.37 |
| Phe1-NH | 8.29 | - |
| Phe1-α | 4.34 | 55.11 |
| Phe1-β | 2.97/2.83 | 36.37 |
| Phe1-γ | - | 137.37 |
| Phe1-δ | 7.29 | 129.16 |
| Phe1-ε | 7.29 | 128.09 |
| Phe1-ξ | 7.22 | 126.39 |
| Phe1-C' | - | 172.41 |
| Aib2-NH | 8.59 | - |
| Aib2-α | - | 55.77 |
| Aib2-β | 1.28 | 23.57 |
| Aib2-β' | 1.27 | 25.37 |
| Aib2-C' | - | 174.92 |
| Aib3-NH | 7.64 | - |
| Aib3-α | - | 55.86 |
| Aib3-β | 1.32 | 24.04 |
| Aib3-β' | 1.29 | 24.62 |
| Aib3-C' | - | 175.11 |
| Aib4-NH | 7.69 | - |
| Aib4-α | - | 55.86 |
| Aib4-β | 1.39 | 24.99 |
| Aib4-β' | 1.38 | 24.55 |
| Aib4-C' | - | 175.62 |
| Aib5-NH | 7.57 | - |
| Aib5-α | - | 55.92 |
| Aib5-β | 1.41 | 24.99 |
| Aib5-β' | 1.37 | 24.49 |
| Aib5-C' | - | 175.62 |
| Gly6-NH | 7.98 | - |
| Gly6-α | 3.76/3.64 | 43.43 |
| Gly6-C' | - | 170.69 |
| Leu7-NH | 7.74 | - |
| Leu7-α | 4.02 | 53.18 |
| Leu7-β | 1.70/1.53 | 39.40 |
| Leu7-γ | 1.69 | 24.13 |
| Leu7-δ | 0.92 | 22.62 |
| Leu7-δ' | 0.85 | 21.57 |
| Leu7-C' | - | 171.80 |
| Iva8-NH | 7.39 | - |
| Iva8-α | - | 59.34 |
| Iva8-β | 2.20/1.67 | 27.60 |
| Iva8-γ | 0.73 | 7.27 |
| Iva8-αMe | 1.27 | 21.93 |
| Iva8-C' | - | 176.07 |
| Aib9-NH | 7.57 | - |
| Aib9-α | - | 56.22 |
| Aib9-β | 1.48 | 23.17 |
| Aib9-β' | 1.37 | 25.72 |
| Aib9-C' | - | 173.44 |
| Hyp10-α | 4.38 | 61.01 |
| Hyp10-β | 2.16/1.77 | 36.84 |
| Hyp10-γ | 4.29 | 68.95 |
| Hyp10-γOH | 5.11 | - |
| Hyp10-δ | 3.73/3.50 | 56.13 |
| Hyp10-C' | - | 171.80 |
| Gln11-NH | 7.89 | - |
| Gln11-α | 4.17 | 52.42 |
| Gln11-β | 2.16/1.86 | 26.66 |
| Gln11-γ | 2.09 | 31.44 |
| Gln11-δ | - | 173.08 |
| Gln11-ε-NH₂ | 7.19/6.70 | - |
| Gln11-C' | - | 172.13 |
| Iva12-NH | 7.47 | - |
| Iva12-α | - | 58.48 |
| Iva12-β | 2.15/1.78 | 28.06 |
| Iva12-γ | 0.74 | 6.97 |
| Iva12-αMe | 1.41 | 20.36 |
| Iva12-C' | - | 172.83 |
| Hyp13-α | 4.53 | 60.55 |
| Hyp13-β | 2.17/1.67 | 37.33 |
| Hyp13-γ | 4.21 | 69.04 |
| Hyp13-γOH | 5.09 | - |
| Hyp13-δ | 3.67/3.37 | 56.46 |
| Hyp13-C' | - | 172.83 |
| Aib14-NH | 7.94 | - |
| Aib14-α | - | 55.65 |
| Aib14-β | 1.40 | 23.50 |
| Aib14-β' | 1.33 | 25.66 |
| Aib14-C' | - | 171.73 |
| Pro15-α | 4.13 | 61.79 |
| Pro15-β | 1.83/1.16 | 28.34 |
| Pro15-γ | 1.58/1.48 | 24.83 |
| Pro15-δ | 3.79/3.50 | 47.36 |
| Pro15-C' | - | 170.69 |
| Phe16-NH | 7.15 | - |
| Phe16-α | 3.84 | 52.60 |
| Phe16-β | 2.99/2.57 | 36.37 |
| Phe16-γ | - | 139.47 |
| Phe16-δ | 7.25 | 129.33 |
| Phe16-ε | 7.24 | 127.93 |
| Phe16-ξ | 7.13 | 125.65 |
| Phe16-CH₂-OH | 3.39/3.24 | 63.42 |
| Phe16-CH₂-OH | 4.57 | - |

**Tabelle 1a:**

| NMR Daten (chemische Verschiebungen) von Cephaibol A1 in DMSO bei 300° K. | | |
|---|---|---|
| Proton/Kohlenstoff | ¹H | ¹³C |
| | | |
| Ac-Me | 1.83 | 22.34 |
| Ac-C' | - | 170.18 |
| Phe1-NH | 8.22 | - |
| Phe1-α | 4.39 | 54.88 |
| Phe1-β | 3.04/2.83 | 36.41 |
| Phe1-γ | - | 137.53 |
| Phe1-δ | 7.29 | 129.10 |
| Phe1-ε | 7.29 | 128.04 |
| Phe1-ξ | 7.21 | 126.32 |
| Phe1-C' | - | 172.09 |
| Aib2-NH | 8.50 | - |
| Aib2-α | - | 55.73 |
| Aib2-β | 1.31 | 24.39 |
| Aib2-β' | 1.30 | 24.60 |
| Aib2-C' | - | 174.55 |
| Aib3-NH | 7.73 | - |
| Aib3-α | - | 55.73 |
| Aib3-β | 1.32 | 25.41 |
| Aib3-β' | 1.29 | 23.12 |
| Aib3-C' | - | 175.38 |
| Aib4-NH | 7.80 | - |
| Aib4-α | - | 55.82 |
| Aib4-β | 1.35 | 25.92 |
| Aib4-β' | 1.34 | 23.12 |
| Aib4-C' | - | 175.60 |
| Aib5-NH | 7.66 | - |
| Aib5-α | - | 55.70 |
| Aib5-β | 1.40 | 26.24 |
| Aib5-β' | 1.39 | 23.20 |
| Aib5-C' | - | 175.68 |
| Ala6-NH | 7.70 | - |
| Ala6-α | 4.01 | 50.69 |
| Ala6-β | 1.38 | 16.47 |
| Ala6-C' | - | 174.55 |
| Leu7-NH | 7.67 | - |
| Leu7-α | 3.96 | 53.49 |
| Leu7-β | 1.67/1.57 | 39.29 |
| Leu7-γ | 1.72 | 24.18 |
| Leu7-δ | 0.90 | 22.58 |
| Leu7-δ' | 0.84 | 21.29 |
| Leu7-C' | - | 171.81 |
| Iva8-NH | 7.18 | - |
| Iva8-α | - | 59.29 |
| Iva8-β | 2.31/1.65 | 26.62 |
| Iva8-γ | 0.70 | 7.15 |
| Iva8-αMe | 1.28 | 22.37 |
| Iva8-C' | - | 176.12 |
| Aib9-NH | 7.46 | - |
| Aib9-α | - | 56.16 |
| Aib9-β | 1.50 | 23.12 |
| Aib9-β' | 1.38 | 25.65 |
| Aib9-C' | - | 173.39 |
| Hyp10-α | 4.39 | 60.99 |
| Hyp10-β | 2.17/1.78 | 36.95 |
| Hyp10-γ | 4.29 | 68.94 |
| Hyp10-γOH | 5.11 | - |
| Hyp10-δ | 3.74/3.55 | 56.27 |
| Hyp10-C' | - | 171.92 |
| Gln11-NH | 7.91 | - |
| Gln11-α | 4.17 | 52.37 |
| Gln11-β | 2.15/1.86 | 26.71 |
| Gln11-γ | 2.10 | 31.37 |
| Gln11-δ | - | 173.03 |
| Gln11-ε-NH₂ | 7.19/6.70 | - |
| Gln11-C' | - | 172.15 |
| Iva12-NH | 7.47 | - |
| Iva12-α | - | 58.47 |
| Iva12-β | 2.15/1.78 | 28.04 |
| Iva12-γ | 0.74 | 6.94 |
| Iva12-αMe | 1.42 | 20.34 |
| Iva12-C' | - | 172.82 |
| Hyp13-α | 4.54 | 60.54 |
| Hyp13-β | 2.17/1.68 | 37.33 |
| Hyp13-γ | 4.22 | 69.03 |
| Hyp13-γOH | 5.08 | - |
| Hyp13-δ | 3.68/3.38 | 56.45 |
| Hyp13-C' | - | 172.82 |
| Aib14-NH | 7.95 | - |
| Aib14-α | - | 55.63 |
| Aib14-β | 1.39 | 23.50 |
| Aib14-β' | 1.33 | 25.65 |
| Aib14-C' | - | 171.71 |
| Pro15-α | 4.13 | 61.78 |
| Pro15-β | 1.85/1.17 | 28.33 |
| Pro15-γ | 1.59/1.48 | 24.82 |
| Pro15-δ | 3.79/3.51 | 47.35 |
| Pro15-C' | - | 170.68 |
| Phe16-NH | 7.16 | - |
| Phe16-α | 3.84 | 52.59 |
| Phe16-β | 2.99/2.58 | 36.41 |
| Phe16-γ | - | 139.46 |
| Phe16-δ | 7.25 | 129.32 |
| Phe16-ε | 7.25 | 127.92 |
| Phe16-ξ | 7.14 | 125.64 |
| Phe16-CH₂-OH | 3.39/3.25 | 63.41 |
| Phe16-CH₂-OH | 4.56 | - |
| a) Im ¹³C-Spektrum wird eine Signalaufspaltung beobachtet. | | |

**Tabelle2:**

| NMR-Daten (chemische Verschiebungen) des Cephaibols B in DMSO bei 300°K. | | |
|---|---|---|
| Proton/Kohlenstoff | ¹H | ¹³C |
| | | |
| Ac-Me | 1.84 | 22.28 |
| Ac-C' | - | 170.37 |
| Phe1-NH | 8.28 | - |
| Phe1-α | 4.33 | 55.14 |
| Phe1-β | 2.97/2.83 | 36.32 |
| Phe1-γ | - | 137.36 |
| Phe1-δ | 7.29 | 129.14 |
| Phe1-ε | 7.29 | 128.05 |
| Phe1-ξ | 7.22 | 126.37 |
| Phe1-C' | - | 172.40 |
| Aib2-NH | 8.58 | - |
| Aib2-α | - | 55.79 |
| Aib2-β | 1.28 | 23.69 |
| Aib2-β' | 1.27 | 25.24 |
| Aib2-C' | - | 174.81 |
| Aib3-NH | 7.62 | - |
| Aib3-α | - | 55.84 |
| Aib3-β | 1.32 | 24.10 |
| Aib3-β' | 1.29 | 24.60 |
| Aib3-C' | - | 175.10 |
| Aib4-NH | 7.73 | - |
| Aib4-α | - | 55.96 |
| Aib4-β | 1.38 | 24.82 |
| Aib4-β' | 1.38 | 24.82 |
| Aib4-C' | - | 175.45 |
| Iva5-NH | 7.49 | - |
| Iva5-α | - | 58.98 |
| Iva5-β | 1.99/1.70 | ∼28.4(breit) |
| Iva5-γ | 0.80 | 7.49 |
| Iva5-αMe | 1.35 | 21.23 |
| Iva5-C' | - | 175.56 |
| Gly6-NH | 7.95 | - |
| Gly6-α | 3.77/3.64 | 43.34 |
| Gly6-C' | - | 170.67 |
| Leu7-NH | 7.74 | - |
| Leu7-α | 4.02 | 53.06 |
| Leu7-β | 1.69/1.53 | 39.40 |
| Leu7-γ | 1.69 | 24.10 |
| Leu7-δ | 0.92 | 22.63 |
| Leu7-δ' | 0.85 | 21.57 |
| Leu7-C' | - | 171.71 |
| Iva8-NH | 7.41 | - |
| Iva8-α | - | 59.35 |
| Iva8-β | 2.17/1.67 | 27.89 |
| Iva8-γ | 0.74 | 7.32 |
| Iva8-αMe | 1.27 | 21.77 |
| Iva8-C' | - | 176.08 |
| Aib9-NH | 7.58 | - |
| Aib9-α | - | 56.21 |
| Aib9-β | 1.48 | 23.15 |
| Aib9-β' | 1.36 | 25.75 |
| Aib9-C' | - | 173.44 |
| Hyp10-α | 4.39 | 60.99 |
| Hyp10-β | 2.16/1.78 | 36.83, 36.77^{a)} |
| Hyp10-γ | 4.28 | 68.94, 68.84^{a)} |
| Hyp10-γOH | 5.11 | - |
| Hyp10-δ | 3.73/3.50 | 56.09 |
| Hyp10-C' | - | 171.77 |
| Gln11-NH | 7.88 | - |
| Gln11-α | 4.17 | 52.42 |
| Gln11-β | 2.15/1.87 | 26.66 |
| Gln11-γ | 2.10 | 31.42 |
| Gln11-δ | - | 173.00 |
| Gln11-ε-NH₂ | 7.18/6.70 | - |
| Gln11-C' | - | 172.11 |
| Iva12-NH | 7.46 | - |
| Iva12-α | - | 58.47 |
| Iva12-β | 2.15/1.77 | 28.06 |
| Iva12-γ | 0.74 | 6.97 |
| Iva12-αMe | 1.42 | 20.35 |
| Iva12-C' | - | 172.81 |
| Hyp13-α | 4.53 | 60.55 |
| Hyp13-β | 2.18/1.68 | 37.32, 37.26^{a)} |
| Hyp13-γ | 4.21 | 69.03, 68.94^{a)} |
| Hyp13-γOH | 5.08 | - |
| Hyp13-δ | 3.67/3.37 | 56.40 |
| Hyp13-C' | - | 172.81 |
| Aib14-NH | 7.94 | - |
| Aib14-α | - | 55.63 |
| Aib14-β | 1.40 | 23.49 |
| Aib14-β' | 1.33 | 25.65 |
| Aib14-C' | - | 171.71 |
| Pro15-α | 4.13 | 61.78 |
| Pro15-β | 1.83/1.16 | 28.33 |
| Pro15-γ | 1.58/1.47 | 24.82 |
| Pro15-δ | 3.79/3.50 | 47.35 |
| Pro15-C' | - | 170.67 |
| Phe16-NH | 7.15 | - |
| Phe16-α | 3.83 | 52.59, 52.56^{a)} |
| Phe16-β | 2.99/2.57 | 36.37 |
| Phe16-γ | - | 139.46 |
| Phe16-δ | 7.25 | 129.32 |
| Phe16-ε | 7.25 | 127.91 |
| Phe16-ξ | 7.14 | 125.63 |
| Phe16-CH₂-OH | 3.39/3.24 | 63.42, 63.30^{a)} |
| Phe16-CH₂-OH | 4.57 | - |

| | | |
|---|---|---|
| a) Im ¹³C-Spektrum wird eine Signalaufspaltung beobachtet. | | |

**Tabelle 3:**

| NMR-Daten (chemische Verschiebungen) des Cephaibols C in DMSO bei 300° K. | | |
|---|---|---|
| Proton/Kohlenstoff | ¹H | ¹³C |
| | | |
| Ac-Me | 1.83 | 22.23 |
| Ac-C' | - | 170.27 |
| Phe1-NH | 8.27 | - |
| Phe1-α | 4.34 | 54.99 |
| Phe1-β | 2.97/2.83 | 36.34 |
| Phe1-γ | - | 137.35 |
| Phe1-δ | 7.29 | 129.14 |
| Phe1-ε | 7.29 | 128.05 |
| Phe1-ξ | 7.22 | 126.38 |
| Phe1-C' | - | 172.39 |
| Aib2-NH | 8.58 | - |
| Aib2-α | - | 55.75 |
| Aib2-β | 1.28 | 23.51 |
| Aib2-β' | 1.27 | 25.34 |
| Aib2-C' | - | 174.81 |
| Aib3-NH | 7.63 | - |
| Aib3-α | - | 55.87 |
| Aib3-β | 1.32 | 23.97 |
| Aib3-β' | 1.29 | 24.55 |
| Aib3-C' | - | 175.08 |
| Aib4-NH | 7.69 | - |
| Aib4-α | - | 55.87 |
| Aib4-β | 1.38 | 24.85 |
| Aib4-β' | 1.38 | 24.55 |
| Aib4-C' | - | 175.62 |
| Aib5-NH | 7.56 | - |
| Aib5-α | - | 55.87 |
| Aib5-β | 1.41 | 24.85 |
| Atb5-β' | 1.37 | 24.55 |
| Aib5-C' | - | 175.54 |
| Gly6-NH | 7.97 | - |
| Gly6-α | 3.76/3.63 | 43.35 |
| Gly6-C' | - | 170.63 |
| Leu7-NH | 7.72 | - |
| Leu7-α | 4.03 | 53.04 |
| Leu7-β | 1.69/1.51 | 39.41 |
| Leu7-γ | 1.69 | 24.11 |
| Leu7-δ | 0.91 | 22.60 |
| Leu7-δ' | 0.85 | 21.57 |
| Leu7-C' | - | 171.74 |
| Iva8-NH | 7.43 | - |
| Iva8-α | - | 59.34 |
| Iva8-β | 2.17/1.69 | 27.87 |
| Iva8-γ | 0.74 | 7.31 |
| Iva8-αMe | 1.29 | 21.87 |
| Iva8-C' | - | 176.04 |
| Aib9-NH | 7.54 | - |
| Aib9-α | - | 56.21 |
| Aib9-β | 1.48 | 23.23 |
| Aib9-β' | 1.36 | 25.49 |
| Aib9-C' | - | 173.59 |
| Hyp10-α | 4.39 | 61.12 |
| Hyp10-β | 2.16/1.77 | 36.75 |
| Hyp10-γ | 4.20 | 68.93 |
| Hyp10-γOH | Broad | - |
| Hyp10-δ | 3.71/3.50 | 56.21 |
| Hyp10-C' | - | 171.74 |
| Gln11-NH | 7.80 | - |
| Gln11-α | 4.22 | 51.95 |
| Gln11-β | 2.18/1.83 | 26.17 |
| Gln11-γ | 2.09 | 31.23 |
| Gln11-δ | - | 173.10 |
| Gln11-ε-NH₂ | 7.18/6.69 | - |
| Gln11-C' | - | 172.02 |
| Aib12-NH | 7.75 | - |
| Aib12-α | - | 55.87 |
| Aib12-β | 1.50 | 23.80 |
| Aib12-β' | 1.38 | 25.73 |
| Aib12-C' | - | 172.26 |
| Hyp13-α | 4.51 | 60.54 |
| Hyp13-β | 2.16/1.69 | 37.22 |
| Hyp13-γ | 4.21 | 68.99 |
| Hyp13-γOH | Broad | - |
| Hyp13-δ | 3.67/3.34 | 56.32 |
| Hyp13-C' | - | 172.70 |
| Aib14-NH | 7.95 | - |
| Aib14-α | - | 55.65 |
| Aib14-β | 1.41 | 23.41 |
| Aib14-β' | 1.34 | 25.58 |
| Aib14-C' | - | 171.70 |
| Pro15-α | 4.13 | 61.77 |
| Pro15-β | 1.84/1.16 | 28.34 |
| Pro15-γ | 1.58/1.49 | 24.55 |
| Pro15-δ | 3.80/3.51 | 47.36 |
| Pro15-C' | - | 170.63 |
| Phe16-NH | 7.14 | - |
| Phe16-α | 3.83 | 52.46 |
| Phe16-β | 2.98/2.57 | 36.34 |
| Phe16-γ | - | 139.46 |
| Phe16-δ | 7.25 | 129.30 |
| Phe16-ε | 7.25 | 127.90 |
| Phe16-ξ | 7.13 | 125.63 |
| Phe16-CH₂-OH | 3.38/3.24 | 63.32 |
| Phe16-CH₂-OH | Broad | - |

**Tabelle 4:**

| NMR-Daten (chemische Verschiebungen) des Cephaibols D in DMSO bei 300° K. | | |
|---|---|---|
| Proton/Kohlenstoff | ¹H | ¹³C |
| | | |
| Ac-Me | 1.83 | 22.26 |
| Ac-C' | - | 170.27 |
| Phe1-NH | 8.28 | - |
| Phe1-α | 4.34 | 55.05 |
| Phe1-β | 2.97/2.83 | 36.34 |
| Phe1-γ | - | 137.35 |
| Phe1-δ | 7.29 | 129.14 |
| Phe1-ε | 7.29 | 128.05 |
| Phe1-ξ | 7.22 | 126.38 |
| Phe1-C' | - | 172.40 |
| Aib2-NH | 8.59 | - |
| Aib2-α | - | 55.76 |
| Aib2-β | 1.28 | 23.47 |
| Aib2-β' | 1.27 | 25.45 |
| Aib2-C' | - | 174.89 |
| Aib3-NH | 7.62 | - |
| Aib3-α | - | 55.87 |
| Aib3-β | 1.32 | 23.98 |
| Aib3-β' | 1.29 | 24.84 |
| Aib3-C' | - | 175.04 |
| Aib4-NH | 7.69 | - |
| Aib4-α | - | 55.87 |
| Aib4-β | 1.38 | 25.02 |
| Aib4-β' | 1.37 | 24.48 |
| Aib4-C' | - | 175.59 |
| Aib5-NH | 7.57 | - |
| Aib5-α | - | 55.87 |
| Aib5-β | 1.40 | 24.84 |
| Aib5-β' | 1.38 | 24.73 |
| Aib5-C' | - | 175.51 |
| Gly6-NH | 7.98 | - |
| Gly6-α | 3.74/3.63 | 43.28 |
| Gly6-C' | - | 170.35 |
| Leu7-NH | 7.66 | - |
| Leu7-α | 4.05 | 52.56 |
| Leu7-β | 1.66/1.52 | 39.25 |
| Leu7-γ | 1.66 | 24.07 |
| Leu7-δ | 0.91 | 22.67 |
| Leu7-δ' | 0.85 | 21.64 |
| Leu7-C' | - | 171.60 |
| Aib8-NH | 7.78 | - |
| Aib8-α | - | 56.15 |
| Aib8-β | 1.45 | 25.45 |
| Aib8-β' | 1.35 | 25.02 |
| Aib8-C' | - | 175.80 |
| Aib9-NH | 7.52 | - |
| Aib9-α | - | 56.22 |
| Aib9-β | 1.46 | 23.33 |
| Aib9-β' | 1.34 | 25.45 |
| Aib9-C' | - | 173.64 |
| Hyp10-α | 4.38 | 61.09 |
| Hyp10-β | 2.15/1.78 | 36.58 |
| Hyp10-γ | 4.29 | 69.01 |
| Hyp10-γOH | Breit | - |
| Hyp10-δ | 3.75/3.46 | 56.06 |
| Hyp10-C' | - | 171.67 |
| Gln11-NH | 7.77 | - |
| Gln11-α | 4.22 | 51.96 |
| Gln11-β | 2.18/1.84 | 26.16 |
| Gln11-γ | 2.09 | 31.33 |
| Gln11-δ | - | 173.10 |
| Gln11-ε-NH₂ | 7.18/6.68 | - |
| Gln11-C' | - | 172.03 |
| Aib12-NH | 7.76 | - |
| Aib12-α | - | 55.87 |
| Aib12-β | 1.50 | 23.81 |
| Aib12-β' | 1.38 | 25.76 |
| Aib12-C' | - | 172.27 |
| Hyp13-α | 4.51 | 60.54 |
| Hyp13-β | 2.16/1.70 | 37.25 |
| Hyp13-γ | 4.22 | 69.01 |
| Hyp13-γOH | Breit | - |
| Hyp13-δ | 3.67/3.34 | 56.34 |
| Hyp13-C' | - | 172.75 |
| Aib14-NH | 7.95 | - |
| Aib14-α | - | 55.66 |
| Aib14-β | 1.41 | 23.47 |
| Aib14-β' | 1.34 | 25.60 |
| Aib14-C' | - | 171.73 |
| Pro15-α | 4.13 | 61.77 |
| Pro15-β | 1.84/1.117 | 28.35 |
| Pro15-γ | 1.58/1.49 | 24.84 |
| Pro15-δ | 3.80/3.52 | 47.36 |
| Pro15-C' | - | 170.69 |
| Phe16-NH | 7.14 | - |
| Phe16-α | 3.84 | 52.47 |
| Phe16-β | 2.98/2.58 | 36.34 |
| Phe16-γ | - | 139.46 |
| Phe16-δ | 7.25 | 129.31 |
| Phe16-ε | 7.25 | 127.90 |
| Phe16-ξ | 7.13 | 125.63 |
| Phe16-CH₂-OH | 3.39/3.24 | 63.34 |
| Phe16-CH₂-OH | Breit | - |

**Tabelle 5:**

| NMR-Daten (chemische Verschiebungen) von Ceophaibol E in DMSO bei 300° K. | | |
|---|---|---|
| Proton/Kohlenstoff | ¹H | ¹³C |
| | | |
| Ac-Me | 1.83 | 22.27 |
| Ac-C' | - | 170.36 |
| Phe1-NH | 8.28 | - |
| Phe1-α | 4.34 | 55.10 |
| Phe1-β | 2.98/2.84 | 36.35 |
| Phe1-γ | - | 137.36 |
| Phe1-δ | 7.29 | 129.15 |
| Phe1-ε | 7.29 | 128.06 |
| Phe1-ξ | 7.23 | 126.39 |
| Phe1-C' | - | 172.42 |
| Aib2-NH | 8.58 | - |
| Aib2-α | - | 55.76 |
| Aib2-β | 1.28 | 25.39 |
| Aib2-β' | 1.27 | 23.51 |
| Aib2-C' | - | 174.90 |
| Aib3-NH | 7.62 | - |
| Aib3-α | - | 55.87 |
| Aib3-β | 1.32 | 24.08 |
| Aib3-β' | 1.29 | 24.63 |
| Aib3-C' | - | 175.06 |
| Aib4-NH | 7.69 | - |
| Aib4-α | - | 55.87 |
| Aib4-β | 1.38 | 25.14 |
| Aib4-β' | 1.38 | 24.94 |
| Aib4-C' | - | 175.60 |
| Aib5-NH | 7.58 | - |
| Aib5-α | - | 55.87 |
| Aib5-β | 1.40 | 24.94 |
| Aib5-β' | 1.37 | 24.53 |
| Aib5-C' | - | 175.64 |
| Gly6-NH | 7.99 | - |
| Gly6-α | 3.74/3.64 | 43.35 |
| Gly6-C' | - | 170.43 |
| Leu7-NH | 7.68 | - |
| Leu7-α | 4.04 | 52.73 |
| Leu7-β | 1.67/1.54 | 39.24 |
| Leu7-γ | 1.67 | 24.08 |
| Leu7-δ | 0.91 | 22.67 |
| Leu7-δ' | 0.86 | 21.64 |
| Leu7-C' | - | 171.71 |
| Aib8-NH | 7.72 | - |
| Aib8-α | - | 56.18 |
| Aib8-β | 1.45 | 25.26 |
| Aib8-β' | 1.34 | 25.66 |
| Aib8-C' | - | 175.84 |
| Aib9-NH | 7.54 | - |
| Aib9-α | - | 56.22 |
| Aib9-β | 1.46 | 23.26 |
| Aib9-β' | 1.34 | 25.66 |
| Aib9-C' | - | 173.48 |
| Hyp10-α | 4.38 | 60.95 |
| Hyp10-β | 2.16/1.79 | 36.71 |
| Hyp10-γ | 4.29 | 68.95 |
| Hyp10-γOH | Breit | - |
| Hyp10-δ | 3.77/3.48 | 56.00 |
| Hyp10-C' | - | 171.75 |
| Gln11-NH | 7.85 | - |
| Gln11-α | 4.17 | 52.48 |
| Gln11-β | 2.16/1.89 | 26.63 |
| Gln11-γ | 2.11 | 31.49 |
| Gln11-δ | - | 173.08 |
| Gln11-ε-NH₂ | 7.19/6.69 | - |
| Gln11-C' | - | 172.13 |
| Iva12-NH | 7.47 | - |
| Iva12-α | - | 58.47 |
| Iva12-β | 2.15/1.78 | 28.09 |
| Iva12-γ | 0.75 | 7.01 |
| Iva12-αMe | 1.41 | 20.37 |
| Iva12-C' | - | 172.83 |
| Hyp13-α | 4.53 | 60.54 |
| Hyp13-β | 2.17/1.68 | 37.32 |
| Hyp13-γ | 4.21 | 69.00 |
| Hyp13-γOH | Breit | - |
| Hyp13-δ | 3.69/3.38 | 56.45 |
| Hyp13-C' | - | 172.83 |
| Aib14-NH | 7.94 | - |
| Aib14-α | - | 55.64 |
| Aib14-β | 1.39 | 23.51 |
| Aib14-β' | 1.33 | 25.66 |
| Aib14-C' | - | 171.71 |
| Pro15-α | 4.13 | 61.78 |
| Pro15-β | 1.85/1.17 | 28.33 |
| Pro15-γ | 1.59/1.48 | 24.82 |
| Pro15-δ | 3.80/3.51 | 47.36 |
| Pro15-C' | - | 170.68 |
| Phe16-NH | 7.15 | - |
| Phe16-α | 3.84 | 52.58 |
| Phe16-β | 2.98/2.58 | 36.35 |
| Phe16-γ | - | 139.47 |
| Phe16-δ | 7.26 | 129.33 |
| Phe16-ε | 7.26 | 127.92 |
| Phe16-ξ | 7.14 | 125.64 |
| Phe16-CH₂-OH | 3.39/3.25 | 63.38 |
| Phe16-CH₂-OH | Breit | - |

**Tabelle 6:**

| NMR-Daten (chemische Verschiebungen) von Cephaibol P in DMSO bei 300° K. | |
|---|---|
| Proton | ¹H |
| | |
| Ac-Me | 1.83 |
| Ac-C' | - |
| Phe1-NH | 8.23 |
| Phe1-α | 4.48 |
| Phe1-β | 3.05/2.84 |
| Phe1-δ | 7.27 |
| Phe1-ε | 7.27 |
| Phe1-ξ | 7.19 |
| Iva2-NH | 8.28 |
| Iva2-β | 1.86/1.66 |
| Iva2-γ | 0.74 |
| Iva2-αMe | 1.27 |
| Gln3-NH | 8.14 |
| Gln3-α | 3.97 |
| Gln3-β | 1.88 |
| Gln3-γ | 2.18 |
| Gln3-ε-NH₂ | 7.34/6.88 |
| Aib4-NH | 7.99 |
| Aib4-β | 1.39 |
| Aib4-β' | 1.35 |
| Ile5-NH | 7.53 |
| Ile5-α | 3.88 |
| Ile5-β | 1.88 |
| Ile5-γMe | 0.84 |
| Ile5-γ | 1.45/1.18 |
| Ile5-δ | 0.78 |
| Thr6-NH | 7.60 |
| Thr6-α | 3.82 |
| Thr6-β | 4.09 |
| Thr6-γ | 1.12 |
| Thr6-OH | 4.96 |
| Aib7-NH | 7.77 |
| Aib7-β | 1.36 |
| Aib7-β' | 1.36 |
| Leu8-NH | 7.26 |
| Leu8-α | 4.21 |
| Leu8-β | 1.65/1.56 |
| Leu8-γ | 1.68 |
| Leu8-δ | 0.85 |
| Leu8-δ' | 0.78 |
| Aib9-NH | 7.94 |
| Aib9-β | 1.52 |
| Aib9-β' | 1.42 |
| Hyp10-α | 4.39 |
| Hyp10-β | 2.16/1.77 |
| Hyp10-γ | 4.26 |
| Hyp10-γOH | 5.12 |
| Hyp10-δ | 3.76/3.44 |
| Gln11-NH | 7.85 |
| Gln11-α | 4.22 |
| Gln11-β | 1.89 |
| Gln11-γ | 2.14/2.12 |
| Gln11-ε-NH₂ | 7.20/6.70 |
| Aib12-NH | 7.79 |
| Aib12-β | 1.51 |
| Aib12-β' | 1.39 |
| Hyp13-α | 4.53 |
| Hyp13-β | 2.18/1.71 |
| Hyp13-γ | 4.22 |
| Hyp13-γOH | 5.09 |
| Hyp13-δ | 3.67/3.38 |
| Aib14-NH | 8.07 |
| Aib14-β | 1.47 |
| Aib14-β' | 1.38 |
| Pro15-α | 4.17 |
| Pro15-β | 1.90/1.04 |
| Pro15-γ | 1.63/1.51 |
| Pro15-δ | 3.87/3.58 |
| Pro15-C' | - |
| Phe16-NH | 7.67 |
| Phe16-α | 4.35 |
| Phe16-β | 3.26/2.80 |
| Phe16-8 | 7.32 |
| Phe16-ε | 7.26 |
| Phe16-ξ | 7.19 |
| Ser17-NH | 7.39 |
| Ser17-α | 4.24 |
| Ser17-β | 3.71 |
| Ser17-βOH | 4.77(breit) |

**Tabelle 7:**

| NMR-Daten (chemische Verschiebungen) von Cephaibol Q in DMSO bei 300° K. | |
|---|---|
| Proton | ¹H |
| | |
| Ac-Me | 1.83 |
| Ac-C' | - |
| Phe1-NH | 8.23 |
| Phe1-α | 4.48 |
| Phe1-β | 3.05/2.84 |
| Phe1-δ | 7.27 |
| Phe1-ε | 7.27 |
| Phe1-ξ | 7.19 |
| Iva2-NH | 8.28 |
| Iva2-β | 1.86/1.66 |
| Iva2-γ | 0.74 |
| Iva2-αMe | 1.27 |
| Gln3-NH | 8.14 |
| Gln3-α | 3.97 |
| Gln3-β | 1.88 |
| Gln3-γ | 2.18 |
| Gln3-ε-NH₂ | 7.34/6.88 |
| Aib4-NH | 7.99 |
| Aib4-β | 1.39 |
| Aib4-β' | 1.35 |
| Ile5-NH | 7.53 |
| Ile5-α | 3.88 |
| Ile5-β | 1.88 |
| Ile5-γMe | 0.84 |
| Ile5-γ | 1.45/1.18 |
| Ile5-δ | 0.78 |
| Thr6-NH | 7.60 |
| Thr6-α | 3.83 |
| Thr6-β | 4.09 |
| Thr6-γ | 1.11 |
| Thr6-OH | 4.96 |
| Aib7-NH | 7.74 |
| Aib7-β | 1.36 |
| Aib7-β' | 1.36 |
| Leu8-NH | 7.28 |
| Leu8-α | 4.16 |
| Leu8-β | 1.65/1.56 |
| Leu8-γ | 1.69 |
| Leu8-δ | 0.85 |
| Leu8-δ' | 0.78 |
| Aib9-NH | 7.97 |
| Aib9-β | 1.50 |
| Aib9-β' | 1.42 |
| Pro10-α | 4.30 |
| Pro10-β | 2.22/1.69 |
| Pro10-γ | 1.84 |
| Pro10-δ | 3.75/3.53 |
| Gln11-NH | 7.79 |
| Gln11-α | 4.24 |
| Gln11-β | 1.88 |
| Gln11-γ | 2.14/2.12 |
| Gln11-ε-NH₂ | 7.19/6.71 |
| Aib12-NH | 7.78 |
| Aib12-β | 1.50 |
| Aib12-β' | 1.39 |
| Hyp13-α | 4.53 |
| Hyp13-β | 2.18/1.71 |
| Hyp13-γ | 4.22 |
| Hyp13-γOH | 5.09 |
| Hyp13-δ | 3.67/3.38 |
| Aib14-NH | 8.07 |
| Aib14-β | 1.47 |
| Aib14-β' | 1.38 |
| Pro15-α | 4.17 |
| Pro15-β | 1.90/1.04 |
| Pro15-γ | 1.63/1.51 |
| Pro15-δ | 3.87/3.58 |
| Pro15-C' | - |
| Phe16-NH | 7.67 |
| Phe16-α | 4.35 |
| Phe16-β | 3.26/2.80 |
| Phe16-δ | 7.32 |
| Phe16-ε | 7.26 |
| Phe16-ξ | 7.19 |
| Ser17-NH | 7.39 |
| Ser17-α | 4.24 |
| Ser17-β | 3.71 |
| Ser17-βOH | 4.77(breit) |

### Beispiel 10

### Bestimmung der anthelminthischen Wirkung

Zur Prüfung der Wirkung der Cephaibole an Helminthen wurde ein in vitro-Test (Larvenentwicklungstest) mit Larven des Hühnerspulwurms Ascaridia galli durchgeführt. Embryonierte Eier von A.galli wurden durch Behandlung mit 5% iger Natriumhypochloritlösung oberflächensterilisiert und nach Entfernung dieser Lösung durch rotierende Glasperlen (5 mm) mechanisch geöffnet. Die geschlüpften L2-Larven wurden via Larvenanreicherungsverfahren angereichert, in einem Nährmedium aufgenommen und bei 41°C und 10% CO₂ inkubiert. 48 Stunden nach dem Schlupf wurden die Larven in Mikrotiterplatten (96 well) 5 Tage mit medikiertem Medium (200 µl) in den Konzentrationen (200,100,50....0.1 µg/ml) inkubiert (41°C, 10% CO₂). Während der 5-tägigen Inkubationszeit wurden Motilität, Morphologie und Vitalität täglich mikroskopisch kontrolliert und dokumentiert. Nach Medikation wurde die Larvenkultur 48 Stunden mit Neutralrot in einer Konzentration von 0.16% inkubiert; nach Entfernung des Neutralrots durch Mediumwechsel wurde die Anreicherung des Vitatfarbstoffes im Darm der Larven als Indiz für eine aktive Nahrungsaufnahme und damit als Nachweis der Vitalität bewertet. Die Medikation der Larven mit Cephaibol A führte in den Konzentrationen 200,100,50 und 25 µg/ml zu 100% Mortalität; bei einer Konzentration von 12.5µg/ml und 6.25 µg/ml betrug die Mortalität 92% bzw. 20%.

### Beispiel 11

### Wirkung auf Ektoparasiten

Die Wirkung gegen Ektoparasiten wurde im Flohlarventest (Katzenfloh = Ctenocephalides felis) überprüft. 5mg Cephaibol A wurden in 0.5 ml Aceton gelöst und in 500mg Blutmehl (defibriniertes Schafblut) eingemischt. Nachdem das Lösungsmittel verdampft war, wurden jeweils 2g Quarzsand mit medikiertem Blutmehl so vermischt, daß Präparatkonzentrationen von 2000, 1000, 500, 250 ppm etc. resultierten. In das Gemisch aus medikiertem Blutmehl und Quarzsand wurde je medikierter Probe bzw. Lösungsmittelkontrolle 15 Floheier zugegeben, die anschließend bei 37°C und hoher Luftfeuchte inkubiert wurden. Die larvale Entwicklung, Verpuppung und Entwicklung zum adulten Stadium wurde im Abstand von 3-5 Tagen kontrolliert und die Mortalitätsraten dokumentiert. In Abhängigkeit von der Dosis konnte eine larvicide Wirkung von > 90% im Vergleich zur Lösungsmittelkontrolle beobachtet werden.

### Beispiel 12

### Bestimmung der antimikrobiellen Wirkung

Vom antimikrobiellen Spektrum zeigt die Tabelle 8 einige minimalen Hemmkonzentrationen (MHK).

**Tabelle 8:**

| Die minimalen Hemmkonzentrationen (MIC) des Cephaibols A gegen einige ausgewählte Mikroorganismen. | |
|---|---|
| Stamm | MIC [µg/ml] |
| Enterococcus hirae ATCC 10541 | 64 |
| Enterococcus faecalis ATCC 29212 | 64 |
| Enterobacter cloacae P 99 | > 128 |
| Enterobacter cloacae 1321 E | > 128 |
| Escherichia coli TEM | > 128 |
| Escherichia coli 1507 E | > 128 |
| Escherichia coli DC 0 | > 128 |
| Escherichia coli DC 2 | > 128 |
| Escherichia coli ATCC 25922 | > 128 |
| Klebsiella aerogenes 1082 E | > 128 |
| Klebsiella oxytoca 1522 E | > 128 |
| Pseudomonas aeruginosa 77/2 | > 128 |
| Pseudomonas aeruginosa 1771 | > 128 |
| Pseudomonas aeruginosa 1771 M | > 128 |
| Pseudomonas aeruginosa ATCC 9027 | > 128 |
| Pseudomonas aeruginosa ATCC 27853 | > 128 |
| Staphylococcus aureus 285 | 8 |
| Staphylococcus aureus ATCC 29213 | 16 |
| Streptococcus pyogenes 308 A | 32 |
| Streptococcus pyogenes 77 A | > 128 |
| Bordetella bronchiseptica ATCC 4617 | > 128 |
| Pasteurella multocida 3;tox.pos. | > 128 |
| Pasteurella haemolytica 7 | > 128 |
| Bordetella bronchiseptica 9 | > 128 |
| Bordetella bronchiseptica 10 | > 128 |
| Pasteurella multocida P 48 | > 128 |
| Pasteurella haemolytica P 53 | > 128 |
| Mycoplasma gallisepticum | 64 |
| Mycoplasma mycoides LC | 128 |
| Mycoplasma mycoides LC | 64 |

## Patentansprüche

1. Verbindung der Formel I, in der R Phe-ol bedeutet und w, x, y, und z folgende Bedeutung haben:
a) w gleich Gly oder Ala; x gleich Aib und y und z gleich Iva;
b) w gleich Gly; x, y und z gleich Iva;
c) w gleich Gly; x und z gleich Aib und y gleich Iva;
d) w gleich Gly; x, y und z gleich Aib; oder
e) w gleich Gly; x und y gleich Aib und z gleich Iva;
oder Verbindung der Formel II in der x Hyp oder Pro bedeutet,
sowie deren physiologisch verträglichen Salze.

2. Verbindung der Formel I und II gemäß Anspruch 1, **dadurch gekennzeichnet, dass** diese oder sind, sowie deren physiologisch verträglichen Salze.

3. Verfahren zur Herstellung einer Verbindung der Formel I oder II oder ein physiologisch verträgliches Salz davon gemäß einem oder mehreren der Ansprüche 1-2, **dadurch gekennzeichnet, daß** der Mikroorganismus Acremonium tubakii FH 1685 DSM 12774 oder einer seiner Varianten oder Mutanten unter geeigneten Bedingungen in einem Kulturmedium fermentiert wird, bis sich eine oder mehrere Verbindungen der allgemeinen Formel I oder II in dem Kulturmedium anhäufen und anschließend aus dem Kulturmedium isoliert und gegebenenfalls in physiologisch verträglichen Salze überführt werden.

4. Verfahren gemäß Anspruch 3, **dadurch gekennzeichnet, daß** die Fermentation unter aeroben Bedingungen bei einer Temperatur zwischen 18 und 35□C und bei einem pH zwischen 6 und 8 durchgeführt wird.

5. Verbindung der Formel I oder II oder ein physiologisch verträgliches Salz davon gemäß einem oder mehreren der Ansprüche 1-2 zur Verwendung als Arzneimittel.

6. Verbindung der Formel I oder II oder ein physiologisch verträgliches Salz davon gemäß einem oder mehreren der Ansprüche 1-2 zur Verwendung als Antibiotikum.

7. Verbindung der Formel I oder II oder ein physiologisch verträgliches Salz davon gemäß einem oder mehreren der Ansprüche 1-2 zur Verwendung als Antibiotikum gegen menschliche und/oder tierische Parasiten.

8. Verbindung der Formel I oder II oder ein physiologisch verträgliches Salz davon gemäß einem oder mehreren der Ansprüche 1-2 zur Verwendung als Antibiotikum gegen humanpathogene und/oder tierpathogene Endoparasiten und/oder Ektoparasiten.

9. Verbindung der Formel I oder II oder ein physiologisch verträgliches Salz davon gemäß einem oder mehreren der Ansprüche 1-2 zur Verwendung als Anthelminthikum.

10. Verbindung der Formel I oder II oder ein physiologisch verträgliches Salz davon gemäß einem oder mehreren der Ansprüche 1-2 zur Verwendung als Antibiotikum gegen humanpathogene und/oder tierpathogene Trematoden und/oder Nematoden oder gegen humanpathogene und/oder tierpathogene Ektoparasiten, die zur Klasse der Arachidna oder Insecta zählen.

11. Verbindung der Formel I oder II oder ein physiologisch verträgliches Salz davon gemäß einem oder mehreren der Ansprüche 1-2 zur Verwendung als Antimykotikum, Antiprotozoikum oder als antimikrobiell wirksame Substanz.

12. Verbindung der Formel I oder II oder ein physiologisch verträgliches Salz davon gemäß einem oder mehreren der Ansprüche 1-2 zur Behandlung und/oder Prophylaxe von bakteriellen und/oder parasitären Erkrankungen sowie von Pilzinfektionen.

13. Verbindung der Formel I oder II oder ein physiologisch verträgliches Salz davon gemäß einem oder mehreren der Ansprüche 1-2 zur Verwendung als Wachstumsförderer bei Nutztieren sowie Verbesserung der Futterverwertung bei Nutztieren.

14. Pharmazeutische Zubereitung mit einem Gehalt an mindestens einer Verbindung der Formel I oder II oder ein physiologisch verträgliches Salz davon gemäß einem oder mehreren der Ansprüche 1-2.

15. Verfahren zur Herstellung von pharmazeutischen Zubereitungen gemäß Anspruch 14, **dadurch gekennzeichnet, daß** man mindestens eine Verbindung der Formel I oder II oder ein physiologisch verträgliches Salz davon gemäß einem oder mehreren der Ansprüche 1-2 mit geeigneten Hilfs- und/oder Trägerstoffen in eine geeignete Darreichungsform bringt.

16. Pharmazeutische Zubereitung gemäß Anspruch 14 zu Verwendung als Antibiotikum, insbesondere als Antibiotikum gegen menschliche und/oder tierische Parasiten, gegen humanpathogene und/oder tierpathogene Endoparasiten und/oder Ektoparasiten, gegen humanpathogene und/oder tierpathogene Trematoden und/oder Nematoden oder gegen humanpathogene und/oder tierpathogene Ektoparasiten die zur Klasse der Arachidna oder Insecta zählen, als Anthelminthikum, Antimykotikum, Antiprotozoikum oder zur Behandlung und/oder Prophylaxe von bakteriellen und/oder parasitären Erkrankungen sowie von Pilzinfektionen.

## Claims

1. A compound of the formula I in which R is Phe-ol and w, x, y, and z have the following meanings:
a) w is Gly or Ala; x is Aib and y and z are Iva;
b) w is Gly; x, y and z are Iva;
c) w is Gly; x and z are Aib and y is Iva;
d) w is Gly; x, y and z are Aib; or
e) w is Gly; x and y are Aib and z is Iva;
or a compound of the formula II in which x is Hyp or Pro,
and also physiologically tolerable salts thereof.

2. A compound of the formula I and II according to claim 1, **characterized in that** these are or and also physiologically tolerable salts thereof.

3. A process for the preparation of a compound of the formula I or II or a physiologically tolerable salt thereof as claimed in one or more of claims 1-2, which comprises fermenting the microorganism Acremonium tubakii FH 1685 DSM 12774 or one of its variants or mutants under suitable conditions in a culture medium until one or more compounds of the formula I or II accumulate in the culture medium and then isolating them from the culture medium and, if appropriate, converting them into physiologically tolerable salts.

4. The process as claimed in claim 3, wherein the fermentation is carried out under aerobic conditions at a temperature between 18 and 35°C and at a pH between 6 and 8.

5. A compound of the formula I or II or a physiologically tolerable salt thereof as claimed in one or more of claims 1-2 for use as a pharmaceutical.

6. A compound of the formula I or II or a physiologically tolerable salt thereof as claimed in one or more of claims 1-2 for use as an antibiotic.

7. A compound of the formula I or II or a physiologically tolerable salt thereof as claimed in one or more of claims 1-2 for use as an antibiotic against human and/or animal parasites.

8. A compound of the formula I or II or a physiologically tolerable salt thereof as claimed in one or more of claims 1-2 for use as an antibiotic against human-pathogenic and/or animal-pathogenic endoparasites and/or ectoparasites.

9. A compound of the formula I or II or a physiologically tolerable salt thereof as claimed in one or more of claims 1-2 for use as an anthelmintic.

10. A compound of the formula I or II or a physiologically tolerable salt thereof as claimed in one or more of claims 1-2 for use as an antibiotic against human-pathogenic and/or animal-pathogenic trematodes and/or nematodes or against human-pathogenic and/or animal-pathogenic ectoparasites which belong to the arachnids or insects class.

11. A compound of the formula I or II or a physiologically tolerable salt thereof as claimed in one or more of claims 1-2 for use as an antimycotic, antiprotozoal or as an antimicrobially active substance.

12. A compound of the formula I or II or a physiologically tolerable salt thereof as claimed in one or more of claims 1-2 for the treatment and/or prophylaxis of bacterial and/or parasitic disorders and of fungal infections.

13. A compound of the formula I or II or a physiologically tolerable salt thereof as claimed in one or more of claims 1-2 for use as a growth promoter in farm animals and for the improvement of the feed utilization in farm animals.

14. A pharmaceutical preparation containing at least one compound of the formula I or II or a physiologically tolerable salt thereof as claimed in one or more of claims 1-2.

15. A process for the production of pharmaceutical preparations as claimed in claim 14, which comprises bringing at least one compound of the formula I or II or a physiologically tolerable salt thereof as claimed in one or more of claims 1-2 into a suitable administration form using suitable excipients and/or vehicles.

16. The pharmaceutical preparation as claimed in claim 14 for use as an antibiotic, in particular as an antibiotic against human and/or animal parasites, against human-pathogenic and/or animal-pathogenic endoparasites and/or ectoparasites, against human-pathogenic and/or animal-pathogenic trematodes and/or nematodes or against human-pathogenic and/or animal-pathogenic ectoparasites which belong to the arachnids or insects class, as an anthelmintic, antimycotic, antiprotozoal or for the treatment and/or prophylaxis of bacterial and/or parasitic disorders and of fungal infections.

## Revendications

1. Composé de formule I dans laquelle R signifie Phe-ol et w, x, y et z ont la signification suivante :
a) w représente Gly ou Ala ; x représente Aib et y et z représentent Iva ;
b) w représente Gly ; x, y et z représentent Iva ;
c) w représente Gly ; x et z représentent Aib et y représente Iva ;
d) w représente Gly ; x, y et z représentent Aib ; ou
e) w représente Gly ; x et y représentent Aib et z représente Iva ;
ou composé de formule II dans laquelle x signifie Hyp ou Pro,
ainsi que leurs sels physiologiquement acceptables.

2. Composé de formule I et II selon la revendication 1, **caractérisé en ce qu'**il s'agit de ou ainsi que leurs sels physiologiquement acceptables.

3. Procédé pour la préparation d'un composé de formule I ou II ou un sel physiologiquement acceptable de celui-ci selon l'une ou plusieurs des revendications 1 et 2, **caractérisé en ce que** le microorganisme Acremonium tubakii FH 1685 DSM 12774 ou une de ses variantes ou un de ses mutants est fermenté dans des conditions appropriées dans un milieu de culture jusqu'à l'accumulation d'un ou de plusieurs composés de formule générale I ou II dans le milieu de culture et ceux-ci sont ensuite isolés du milieu de culture et le cas échéant transformés en sels physiologiquement acceptables.

4. Procédé selon la revendication 3, **caractérisé en ce que** la fermentation est réalisée dans des conditions aérobies à une température entre 18 et 35°C et à un pH entre 6 et 8.

5. Composé de formule I ou II ou sel physiologiquement acceptable de celui-ci selon l'une ou plusieurs des revendications 1 et 2 destiné à une utilisation comme médicament.

6. Composé de formule I ou II ou sel physiologiquement acceptable de celui-ci selon l'une ou plusieurs des revendications 1 et 2 destiné à une utilisation comme antibiotique.

7. Composé de formule I ou II ou sel physiologiquement acceptable de celui-ci selon l'une ou plusieurs des revendications 1 et 2. destiné à une utilisation comme antibiotique contre des parasites humains et/ou animaux.

8. Composé de formule I ou II ou sel physiologiquement acceptable de celui-ci selon l'une ou plusieurs des revendications 1 et 2 destiné à une utilisation comme antibiotique contre des endoparasites et/ou des ectoparasites pathogènes pour l'homme et/ou les animaux.

9. Composé de formule I ou II ou sel physiologiquement acceptable de celui-ci selon l'une ou plusieurs des revendications 1 et 2 destiné à une utilisation comme antihelminthique.

10. Composé de formule I ou II ou sel physiologiquement acceptable de celui-ci selon l'une ou plusieurs des revendications 1 et 2 destiné à une utilisation comme antibiotique contre des trématodes et/ou des nématodes pathogènes pour l'homme et/ou les animaux ou contre des ectoparasites pathogènes pour l'homme et/ou les animaux, qui appartiennent à la classe des arachnides ou des insectes.

11. Composé de formule I ou II ou sel physiologiquement acceptable de celui-ci selon l'une ou plusieurs des revendications 1 et 2 destiné à une utilisation comme antimycosique, antiprotozoaire ou comme substance à activité antimicrobienne.

12. Composé de formule I ou II ou sel physiologiquement acceptable de celui-ci selon l'une ou plusieurs des revendications 1 et 2 destiné au traitement et/ou à la prophylaxie de maladies bactériennes et/ou parasitaires ainsi que d'infections par les champignons.

13. Composé de formule I ou II ou sel physiologiquement acceptable de celui-ci selon l'une ou plusieurs des revendications 1 et 2 destiné à une utilisation comme agent promoteur de la croissance pour les animaux d'exploitation ainsi que l'amélioration de l'utilisation du fourrage chez les animaux d'exploitation.

14. Composition pharmaceutique présentant une teneur en au moins un composé de formule I ou II ou un sel physiologiquement acceptable de celui-ci selon l'une ou plusieurs des revendications 1 et 2.

15. Procédé pour la préparation de compositions pharmaceutiques selon la revendication 14, **caractérisé en ce qu'**on amène au moins un composé de formule I ou II ou un sel physiologiquement compatible de celui-ci selon l'une ou plusieurs des revendications 1 et 2 avec des adjuvants et/ou des substances support appropriés dans une forme d'administration appropriée.

16. Composition pharmaceutique selon la revendication 14 destinée à une utilisation comme antibiotique, en particulier comme antibiotique contre des parasites pathogènes pour les hommes et/ou les animaux, des endoparasites et/ou des ectoparasites pathogènes pour l'homme et/ou les animaux, contre les trématodes et/ou les nématodes pathogènes pour l'homme et/ou les animaux ou contre les ectoparasites pathogènes pour l'homme et/ou les animaux qui appartiennent à la classe des arachnides ou des insectes, comme antihelminthique, antimycosique, antiprotozoaire ou au traitement et/ou la prophylaxie de maladies bactériennes et/ou parasitaires ainsi que d'infections par les champignons.


### SEQUENCE LISTING

<110> Aventis Pharma Deutschland GmbH
<120> Cephaibole, neue Antiparasitika aus Acremonium tubakii, Verfahren zu ihrer Herstellung und Verwendung derselben
<130> HMR 1999/L027
<140>
   <141>
<150> 19920816.6
   <151> 1999-05-05
<160> 8
<170> PatencIn Ver. 2.1
<210> 1
   <211> 16
   <212> PRT
   <213> acremonium tubakii
<220>
   <221> PEPTIDE
   <222> (1)
   <223> Xaa = AcPhe
<220>
   <221> PEPTIDE
   <222> (2)..(5)
   <223> Xaa = Aib
<220>
   <221> PEPTIDE
   <222> (8)
   <223> Xaa = Iva
<220>
   <221> PEPTIDE
   <222> (9)
   <223> Xaa = Aib
<220>
   <221> PEPTIDE
   <222> (10)
   <223> Xaa = Hyp
<220>
   <221> PEPTIDE
   <222> (12)
   <223> Xaa = Iva
<220>
   <221> PEPTIDE
   <222> (13)
   <223> Xaa= Hyp
<220>
   <221> PEPTIDE
   <222> (14)
   <223> Xaa = Aib
<220>
   <221> PEPTIDE
   <222> (16)
   <223> Xaa = Phe-ol
<400> 1
   Xaa Xaa Xaa Xaa Xaa Gly Leu Xaa Xaa Xaa Gln Xaa Xaa Xaa Pro Xaa
   1               5                   10                  15
<210> 2
   <211> 16
   <212> PRT
   <213> acremonium tubakii
<220>
   <221> PEPTIDE
   <222> (1)
   <223> Xaa = AcPhe
<220>
   <221> PEPTIDE
   <222> (2)..(4)
   <223> Xaa = Aib
<220>
   <221> PEPTIDE
   <222> (5)
   <223> Xaa = Iva
<220>
   <221> PEPTIDE
   <222> (8)
   <223> Xaa = Iva
<220>
   <221> PEPTIDE
   <222> (9)
   <223> Xaa = Aib
<220>
   <221> PEPTIDE
   <222> (10)
   <223> Xaa = Hyp
<220>
   <221> PEPTIDE
   <222> (12)
   <223> Xaa = Iva
<220>
   <221> PEPTIDE
   <222> (13)
   <223> Xaa = Hyp
<220>
   <221> PEPTIDE
   <222> (14)
   <223> Xaa = Aib
<220>
   <221> PEPTIDE
   <222> (16)
   <223> Xaa = Phe-ol
<400> 2
   Xaa Xaa Xaa Xaa Xaa Gly Leu Xaa Xaa Xaa Gln Xaa Xaa Xaa Pro Xaa
    1               5                  10                  15
<210> 3
   <211> 16
   <212> PRT
   <213> acremonium tubakii
<220>
   <221> PEPTIDE
   <222> (1)
   <223> Xaa = AcPhe
<220>
   <221> PEPTIDE
   <222> (2)..(5)
   <223> Xaa = Aib
<220>
   <221> PEPTIDE
   <222> (8)
   <223> Xaa = Iva
<220>
   <221> PEPTIDE
   <222> (9)
   <223> Xaa = Aib
<220>
   <221> PEPTIDE
   <222> (10)
   <223> Xaa = Hyp
<220>
   <221> PEPTIDE
   <222> (12)
   <223> Xaa = Aib
<220>
   <221> PEPTIDE
   <222> (13)
   <223> Xaa = Hyp
<220>
   <221> PEPTIDE
   <222> (14)
   <223> Xaa = Aib
<220>
   <221> PEPTIDE
   <222> (16)
   <223> Xaa = Phe-ol
<400> 3
   Xaa Xaa Xaa Xaa Xaa Gly Leu Xaa Xaa Xaa Gln Xaa Xaa Xaa Pro Xaa
    1               5                   10                  15
<210> 4
   <211> 16
   <212> PRT
   <213> acremonium tubakii
<220>
   <221> PEPTIDE
   <222> (1)
   <223> Xaa = AcPhe
<220>
   <221> PEPTIDE
   <222> (2)..(5)
   <223> Xaa = Aib
<220>
   <221> PEPTIDE
   <222> (8)..(9)
   <223> Xaa = Aib
<220>
   <221> PEPTIDE
   <222> (10)
   <223> Xaa = Hyp
<220>
   <221> PEPTIDE
   <222> (12)
   <223> Xaa = Aib
<220>
   <221> PEPTIDE
   <222> (13)
   <223> Xaa = Hyp
<220>
   <221> PEPTIDE
   <222> (14)
   <223> Xaa = Aib
<220>
   <221> PEPTIDE
   <222> (16)
   <223> Xaa = Phe-ol
<400> 4
   Xaa Xaa Xaa Xaa Xaa Gly Leu Xaa Xaa Xaa Gln Xaa Xaa Xaa Pro Xaa
   1               5                   10                  15
<210> 5
   <211> 16
   <212> PRT
   <213> acremonium tubakii
<220>
   <221> PEPTIDE
   <222> (1)
   <223> Xaa = AcPhe
<220>
   <221> PEPTIDE
   <222> (2)..(5)
   <223> Xaa = Aib
<220>
   <221> PEPTIDE
   <222> (8)..(9)
   <223> Xaa = Aib
<220>
   <221> PEPTIDE
   <222> (10)
   <223> Xaa = Hyp
<220>
   <221> PEPTIDE
   <222> (12)
   <223> Xaa = Iva
<220>
   <221> PEPTIDE
   <222> (13)
   <223> Xaa = Hyp
<220>
   <221> PEPTIDE
   <222> (14)
   <223> Xaa = Aib
<220>
   <221> PEPTIDE
   <222> (16)
   <223> Xaa = Phe-ol
<400> 5
   Xaa Xaa Xaa Xaa Xaa Gly Leu Xaa Xaa Xaa Gln Xaa Xaa Xaa Pro Xaa
    1               5                   10                  15
<210> 6
   <211> 17
   <212> PRT
   <213> acremonium tubakii
<220>
   <221> PEPTIDE
   <222> (1)
   <223> Xaa = AcPhe
<220>
   <221> PEPTIDE
   <222> (2)
   <223> Xaa = Iva
<220>
   <221> PEPTIDE
   <222> (4)
   <223> Xaa = Aib
<220>
   <221> PEPTIDE
   <222> (7)
   <223> Xaa = Aib
<220>
   <221> PEPTIDE
   <222> (9)
   <223> Xaa = Aib
<220>
   <221> PEPTIDE
   <222> (10)
   <223> Xaa = Hyp
<220>
   <221> PEPTIDE
   <222> (12)
   <223> Xaa = Aib
<220>
   <221> PEPTIDE
   <222> (13)
   <223> Xaa = Hyp
<220>
   <221> PEPTIDE
   <222> (14)
   <223> Xaa = Aib
<400> 6
   Xaa Xaa Gln Xaa Ile Thr Xaa Leu Xaa Xaa Gln Xaa Xaa Xaa Pro Phe
    1               5                  10                  15
   Ser
<210> 7
   <211> 17
   <212> PRT
   <213> acremonium tubakii
<220>
   <221> PEPTIDE
   <222> (1)
   <223> Xaa = AcPhe
<220>
   <221> PEPTIDE
   <222> (2)
   <223> Xaa = Iva
<220>
   <221> PEPTIDE
   <222> (4)
   <223> Xaa = Aib
<220>
   <221> PEPTIDE
   <222> (7)
   <223> Xaa = Aib
<220>
   <221> PEPTIDE
   <222> (9)
   <223> Xaa = Aib
<220>
   <221> PEPTIDE
   <222> (12)
   <223> Xaa = Aib
<220>
   <221> PEPTIDE
   <222> (13)
   <223> Xaa = Hyp
<220>
   <221> PEPTIDE
   <222> (14)
   <223> Xaa = Aib
<400> 7
   Xaa Xaa Gln Xaa Ile Thr Xaa Leu Xaa Pro Gln Xaa Xaa Xaa Pro Phe
    1               5                   10                  15
   Ser
<210> 8
   <211> 16
   <212> PRT
   <213> acremonium tubakii
<220>
   <221> PEPTIDE
   <222> (1)
   <223> Xaa = AcPhe
<220>
   <221> PEPTIDE
   <222> (2)..(5)
   <223> Xaa = Aib
<220>
   <221> PEPTIDE
   <222> (8)
   <223> Xaa = Iva
<220>
   <221> PEPTIDE
   <222> (9)
   <223> Xaa = Aib
<220>
   <221> PEPTIDE
   <222> (10)
   <223> Xaa = Hyp
<220>
   <221> PEPTIDE
   <222> (12)
   <223> Xaa = Iva
<220>
   <221> PEPTIDE
   <222> (13)
   <223> Xaa = Hyp
<220>
   <221> PEPTIDE
   <222> (14)
   <223> Xaa = Aib
<220>
   <221> PEPTIDE
   <222> (16)
   <223> Xaa = Phe-ol
<400> 8
   Xaa Xaa Xaa Xaa Xaa Ala Leu Xaa Xaa Xaa Gln Xaa Xaa Xaa Pro Xaa
    1               5                  10                  15